# EUROPEAN PATENT SPECIFICATION

(11) **EP 3 167 078 B1**
(45) Date of publication and mention of the grant of the patent: **11.09.2019**
(21) Application number: 15745146.9
(22) Date of filing: 09.07.2015
(51) Int. Cl.: C12Q 1/68, C12Q 1/70, C12Q 1/6883

(54) **HCBI, MSBI, MSSI AND CMI SEQUENCES AS AN EARLY MARKER FOR THE FUTURE DEVELOPMENT OF CANCER AND DISEASES OF THE CNS AND AS A TARGET FOR THE TREATMENT AND PREVENTION OF THESE DISEASES**
HCBIO-, MSBI-, MSSI- UND CMI-SEQUENZEN ALS FRÜHE MARKER FÜR DIE ZUKÜNFTIGE ENTWICKLUNG VON KREBS UND ERKRANKUNGEN DES ZNS UND ALS ZIEL ZUR BEHANDLUNG UND PRÄVENTION DIESER ERKRANKUNGEN
SÉQUENCES HCBI, MSBI, MSSI ET CMI UTILISABLES EN TANT QUE MARQUEURS PRÉCOCES DU FUTUR DÉVELOPPEMENT D'UN CANCER ET DE MALADIES DU SNC ET EN TANT QUE CIBLES POUR LE TRAITEMENT ET LA PRÉVENTION DE CES MALADIES

(30) Priority: 10.07.2014 EP 14176624
(43) Date of publication of application: 17.05.2017
(73) Proprietor: Deutsches Krebsforschungszentrum, 69120 Heidelberg (DE)
(72) Inventor: DE VILLIERS-ZUR HAUSEN, Ethel-Michele, 69483 Wald-Michelbach (DE); ZUR HAUSEN, Harald, 69483 Wald-Michelhausen (DE); GUNST, Karin, 69493 Hirschberg (DE); WHITLEY, Corinna, 68519 Vierheim (DE); PÉREZ, Iranzu Lamberto, 31006 Pamplona Navarra (ES)
(74) Representative: Schüssler, Andrea
(86) International application number: PCT/EP2015/001399
(87) International publication number: WO 2016/005054

(56) References cited:
- LAURA MANUELIDIS: "Nuclease resistant circular DNAs copurify with infectivity in scrapie and CJD", JOURNAL OF NEUROVIROLOGY, vol. 17, no. 2, 7 December 2010 (2010-12-07), pages 131-145, XP055158494, ISSN: 1355-0284, DOI: 10.1007/s13365-010-0007-0 & DATABASE EMBL [Online] 10 December 2010 (2010-12-10), "TSE-associated circular DNA isolate Sphinx 1.76, complete sequence.", retrieved from EBI accession no. EM_STD:HQ444404 Database accession no. HQ444404
- EMMANUEL DIAS NETO ET AL: "SHOTGUN SEQUENCING OF THE HUMAN TRANSCRIPTOME WITH ORF EXPRESSED SEQUENCE TAGS", PROCEEDINGS OF THE NATIONAL ACADEMY OF SCIENCES, NATIONAL ACADEMY OF SCIENCES, US, vol. 97, no. 7, 28 March 2000 (2000-03-28) , pages 3491-3496, XP000996193, ISSN: 0027-8424, DOI: 10.1073/PNAS.97.7.3491 & DATABASE EMBL [Online] 17 December 2004 (2004-12-17), "QV0-CT0181-041199-048-g08 CT0181 Homo sapiens cDNA, mRNA sequence.", retrieved from EBI accession no. EM_EST:CK327030 Database accession no. CK327030 & DATABASE EMBL [Online] 20 June 2000 (2000-06-20), "CM2-BT0368-171299-056-b03 BT0368 Homo sapiens cDNA, mRNA sequence.", retrieved from EBI accession no. EM_EST:BE068140 Database accession no. BE068140
- B. PALENIK ET AL: "Coastal Synechococcus metagenome reveals major roles for horizontal gene transfer and plasmids in population diversity", ENVIRONMENTAL MICROBIOLOGY, vol. 11, no. 2, 1 February 2009 (2009-02-01), pages 349-359, XP055158637, ISSN: 1462-2912, DOI: 10.1111/j.1462-2920.2008.01772.x & DATABASE EMBL [Online] 28 September 2011 (2011-09-28), "Uncultured bacterium plasmid clone S6GIO-17 genomic sequence.", retrieved from EBI accession no. EM_STD:JN541288 Database accession no. JN541288

## Description

The present invention relates to MSBI (Multiple Sclerosis Brain Isolate) nucleotide sequences as well as probes and primers comprising part of said nucleotide sequences and antibodies against polypeptides encoded by said nucleotide sequences. Finally, the present invention relates to the use of said compounds as an early marker for the future development of diseases such as Multiple Sclerosis and as a target for treatment and prevention of these diseases.

### Background

Several epidemiological analyses conducted in recent decades indicate that the long-term consumption of "red" meat processed by different ways (including smoked or air-dried meat and meat as component of sausages consumed rare, undercooked or grilled) can be regarded as a risk factor for colon cancer (World Cancer Report 2007, zur Hausen 2012). "Red" meat is regarded as comprising beef, pork, mutton, lamb and goat meat, in contrast to "white" meat (poultry meat/fish).

Thus far, chemical carcinogenic substances being produced during roasting, grilling, barbecuing, smoking and air-drying were blamed as risk factors for cancer. However, often the fact was disregarded that the same substances are also produced in comparable concentrations during analogous ways of preparation of poultry meat/fish. Accordingly, this does not support the assumption that these chemical substances play an exclusive role as regards the development of colon cancer. Since, in addition, the current epidemiological analyses suggest that beef is the main risk factor it has been postulated that an additional species-specific - presumably infectious - factor contributes to the triggering of this type of cancer (zur Hausen, 2012). The results of the correlation of analyses of the global spreading of domesticated bovine species with the global incidence of colon cancer seem to suggest that the consumption of meat of bovine species stemming from European/Asian cattle (Bos taurus) but not from breedings of zebu, water buffalo or yak might be of importance as a main risk factor (zur Hausen, 2015)

Thus, the technical problem underlying the present invention is to identify specific nucleotide sequences that might be associated with diseases such as Multiple Sclerosis and, thus, to provide means for diagnosis and therapy.

### Brief Description of the Present Invention

The solution to said technical problem is achieved by providing the embodiments characterized in the claims.

During the experiments resulting in the present invention sera of cattle were screened for infectious agents - starting from the assumption that the presence in sera is also indicative for the presence of these agents in "red" meat. Sera from healthy cows were screened and new viral nucleic acid components could be isolated. The DNA sequences and open reading frames of several of these components showed a recognizable relationship to two sequences which were already described for transmissible spongiform encephalopathies (TSE) for TSE-diseases of sheep, cattle and humans.

The TSE isolates have also been suspected to play a role in cancer induction (Manuelidis, 2011), thus, it is reasonable to assume that the viral sequences described might be associated with the development of diseases like cancer, specifically colon and breast cancers but also Hodgkin's disease and others, and diseases of the CNS (Multiple sclerosis MS, amyotrophic lateral sclerosis, transmissible spongiforme encephalopathies/ Prion-linked diseases, Parkinson's disease, Alzheimer disease).

### Brief description of the drawings

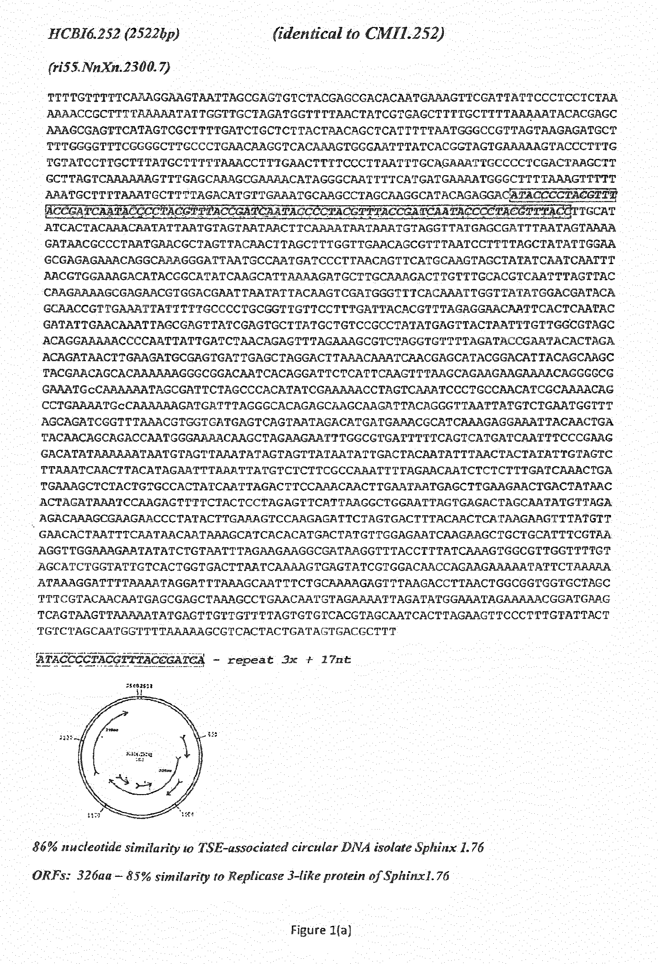

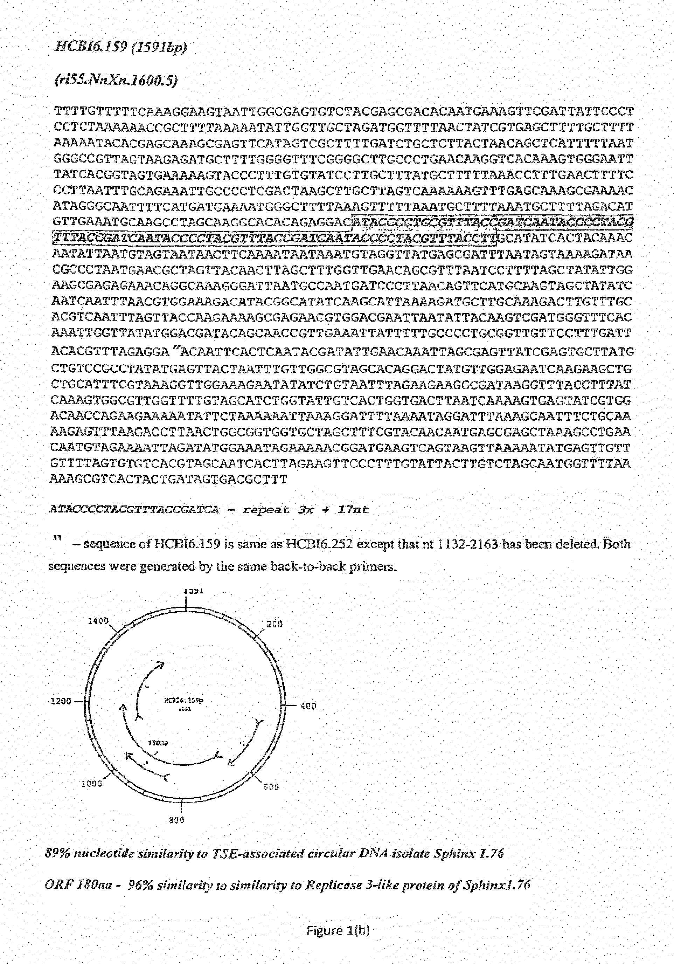

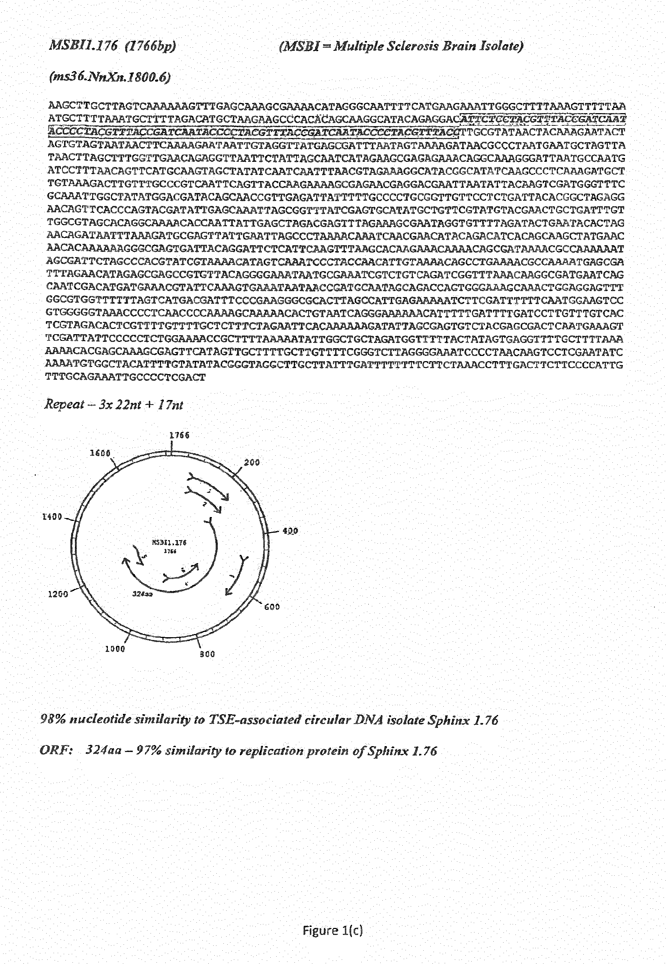

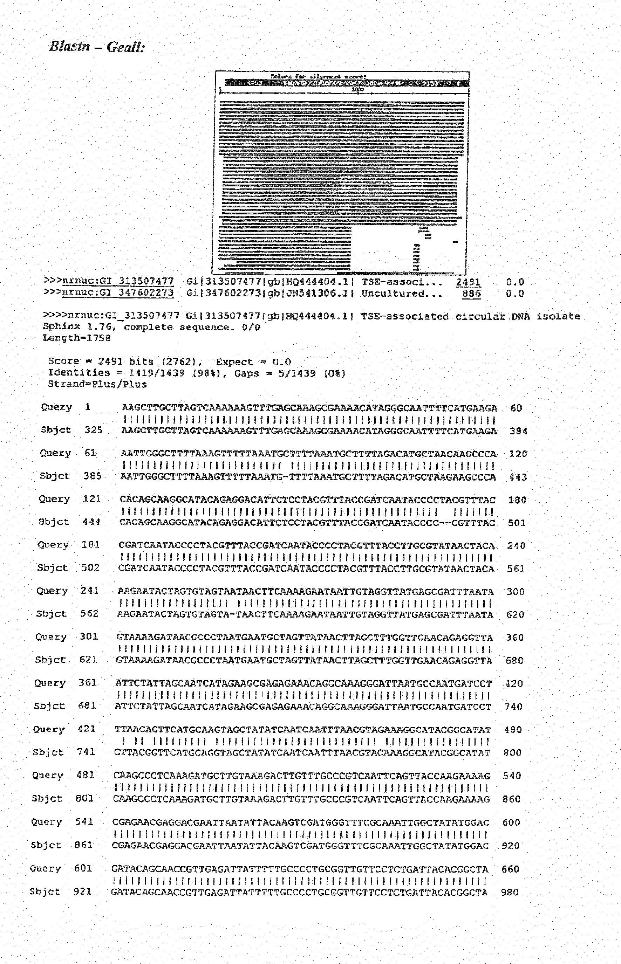

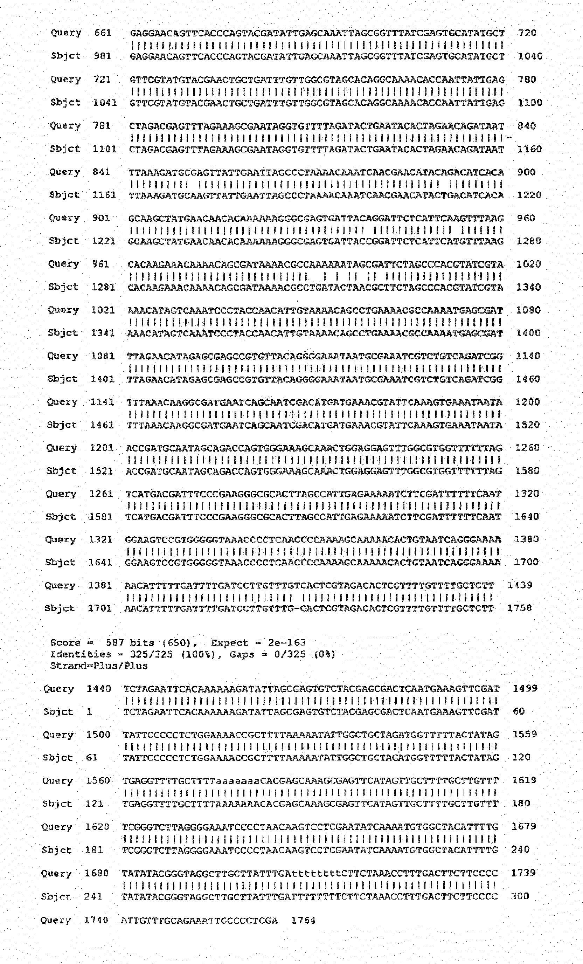

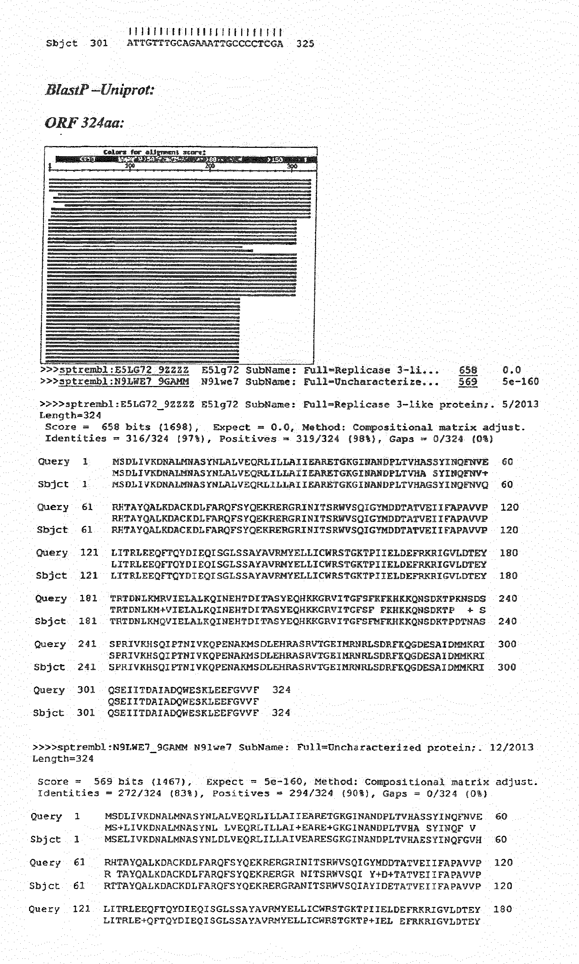

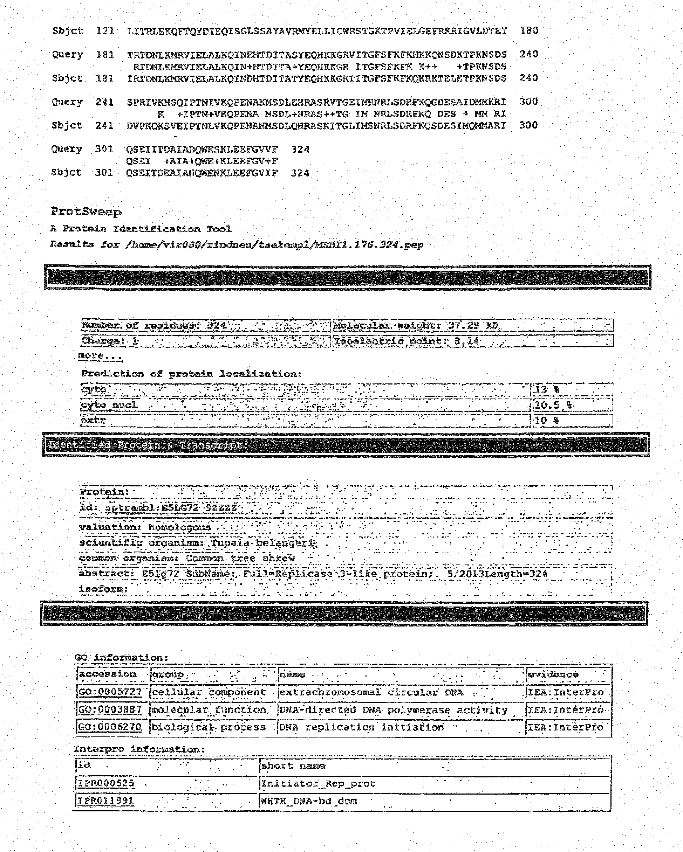

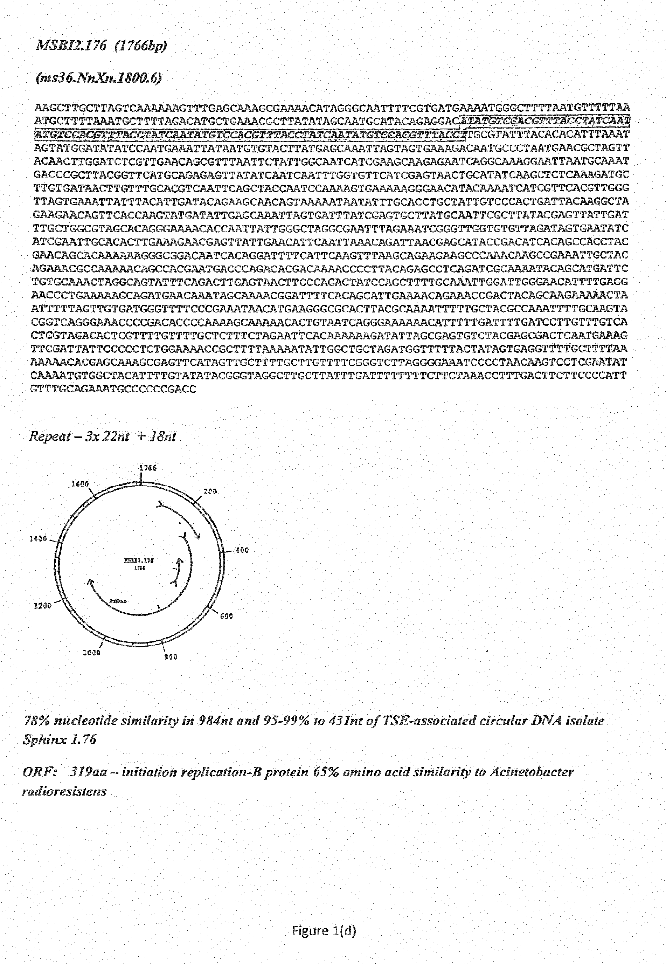

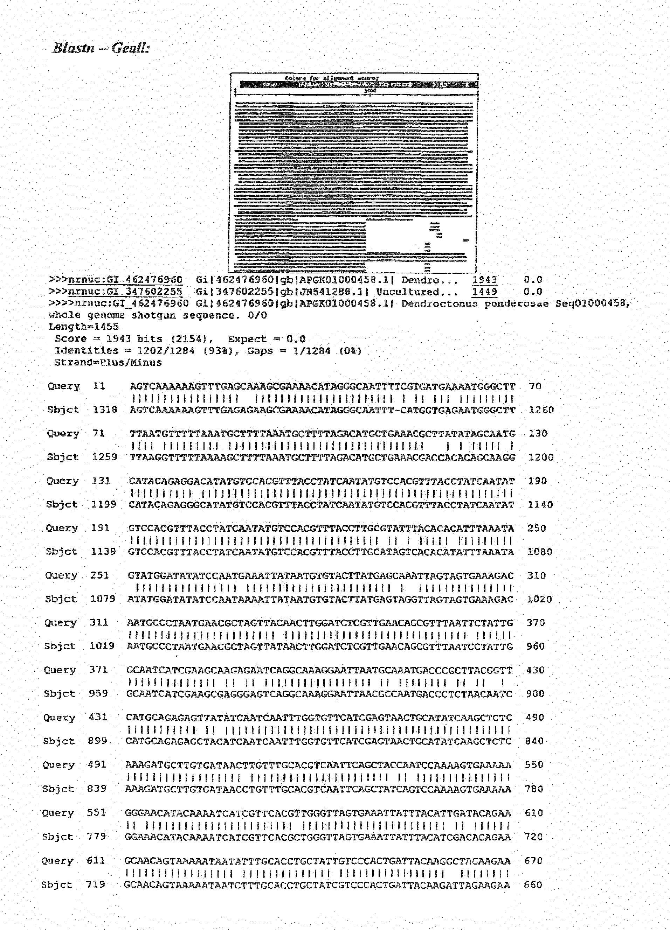

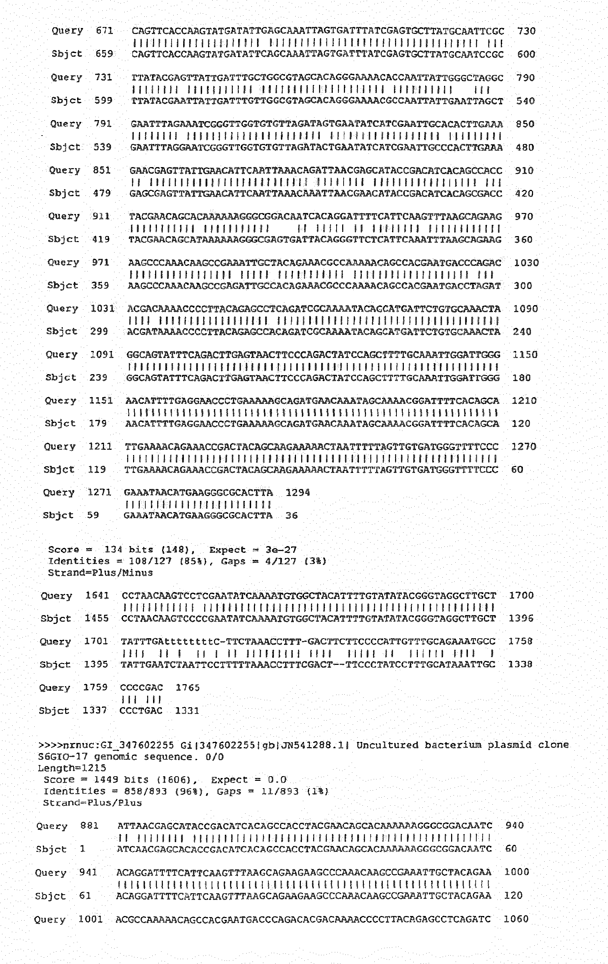

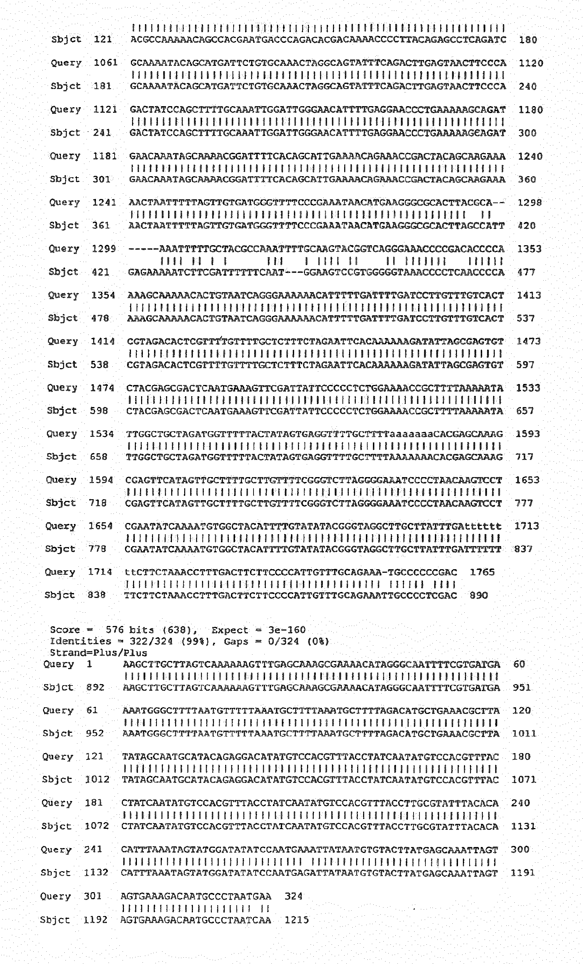

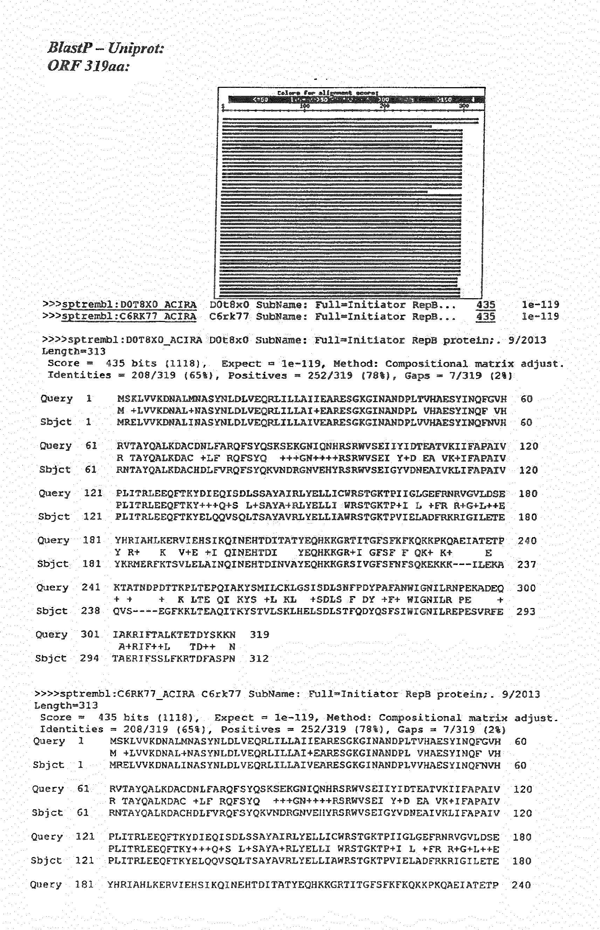

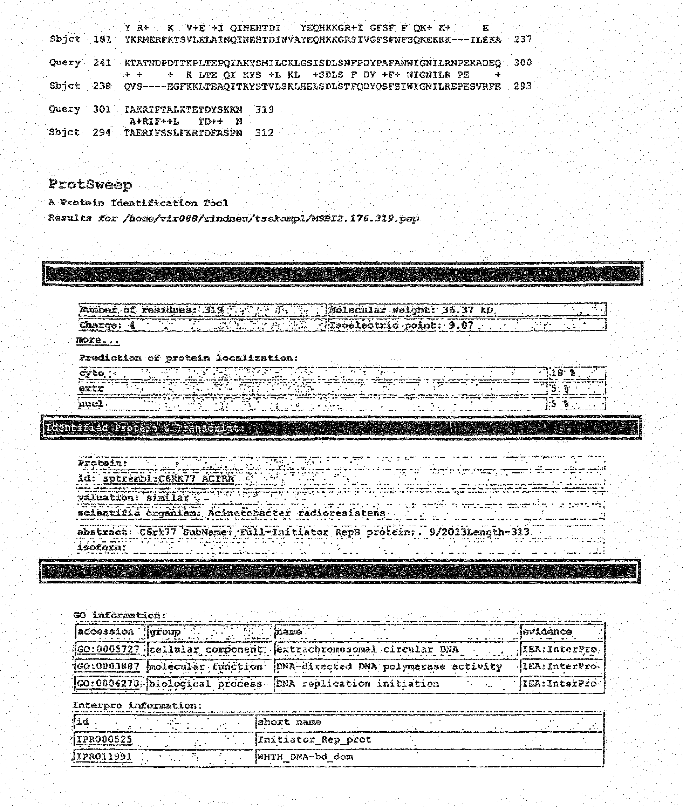

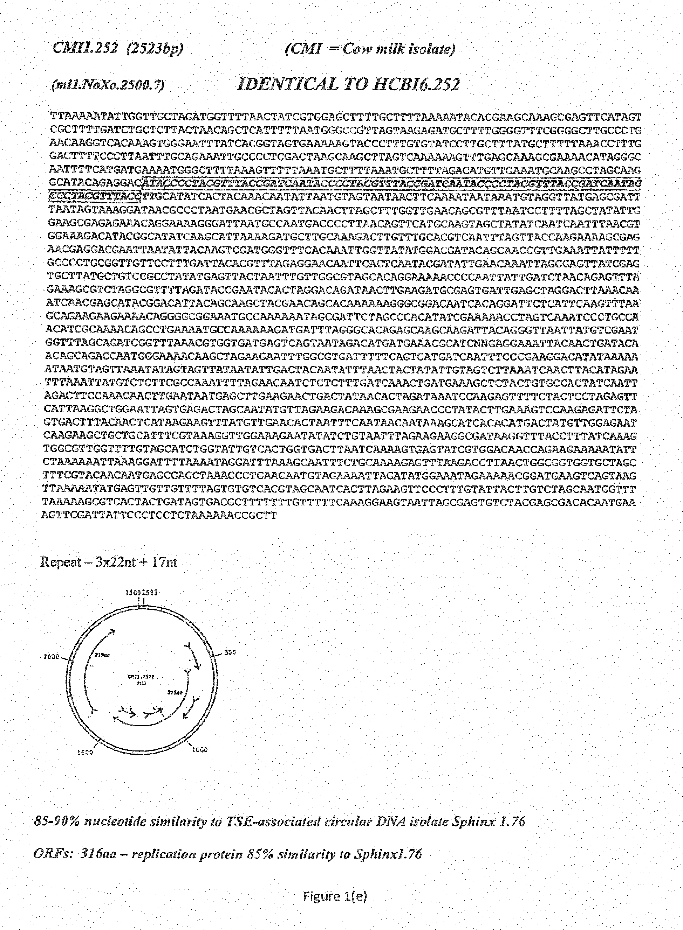

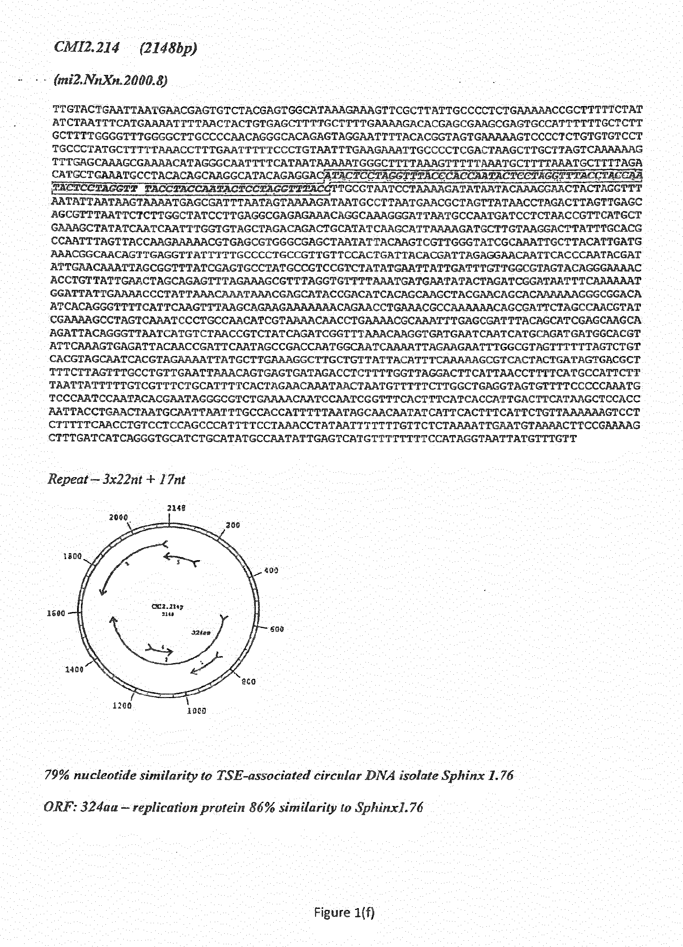

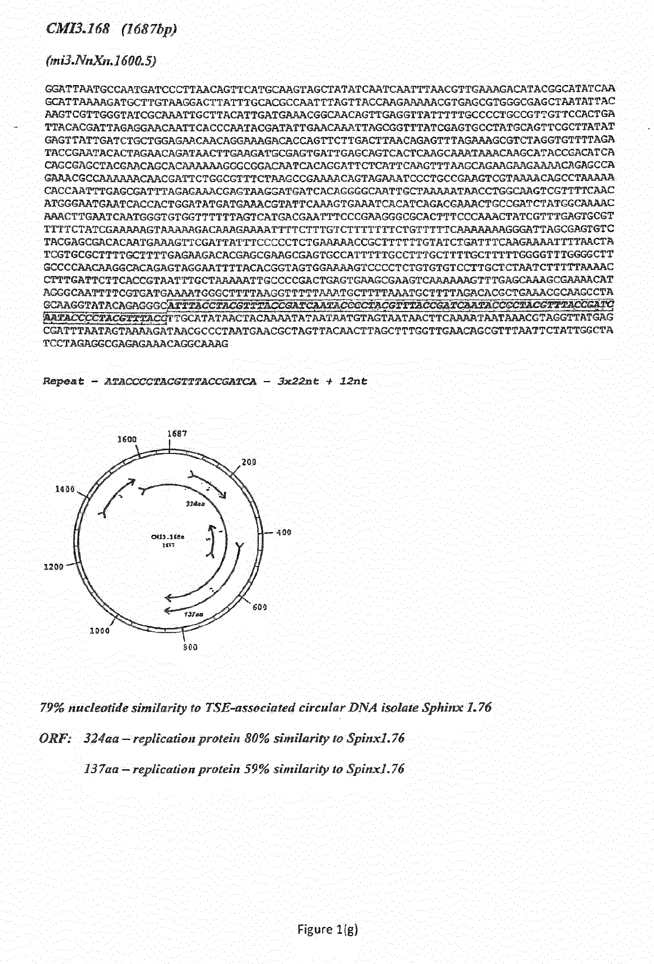

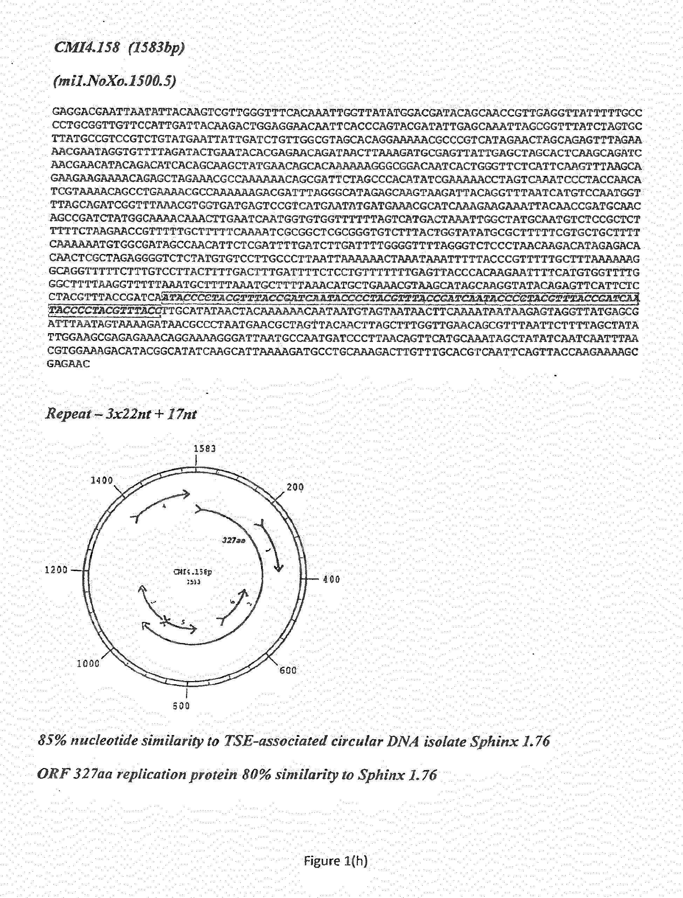

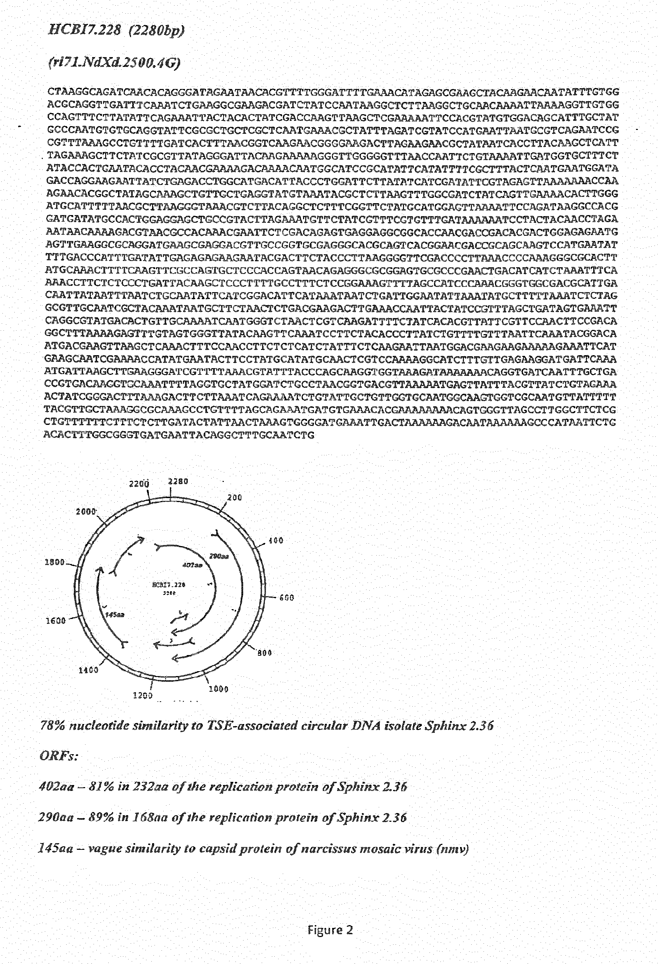

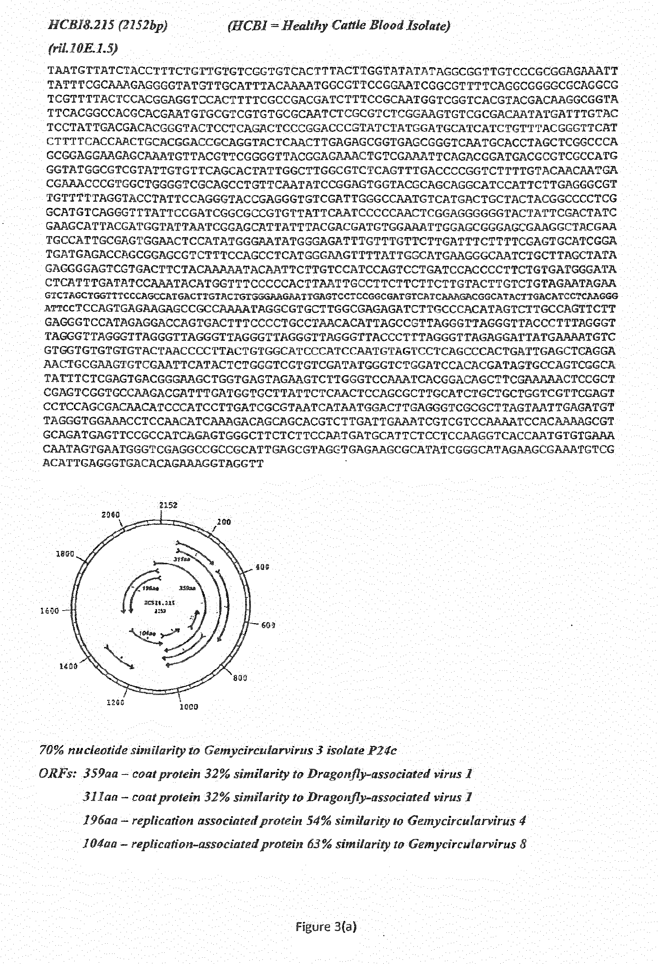

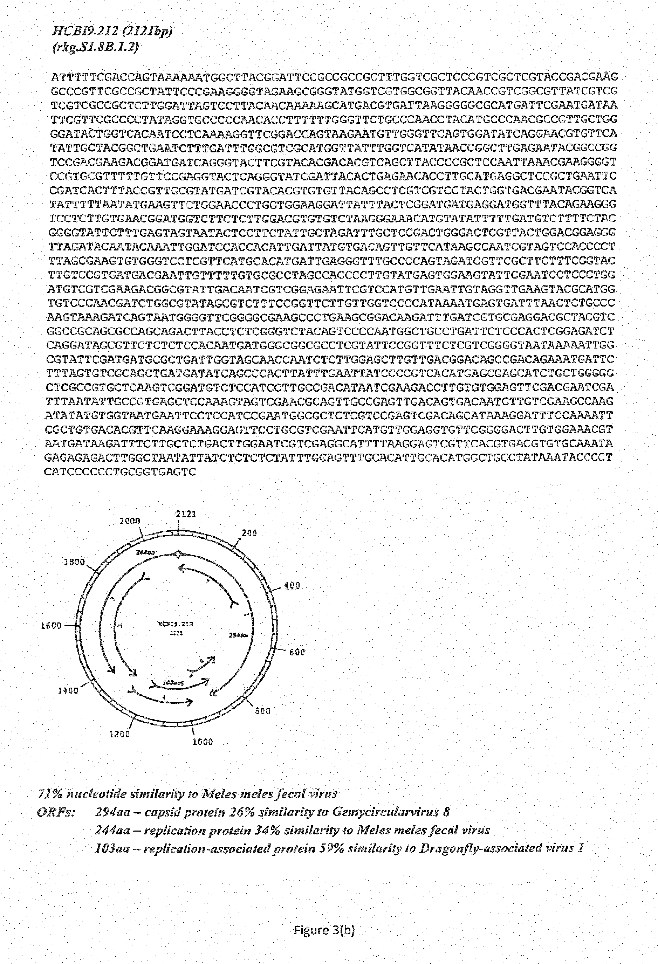

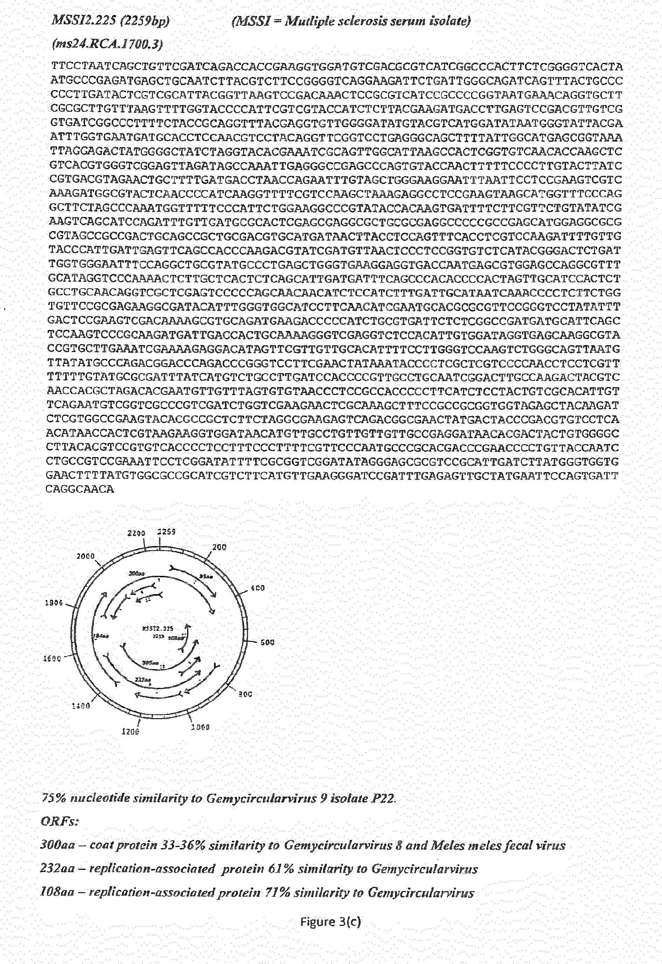

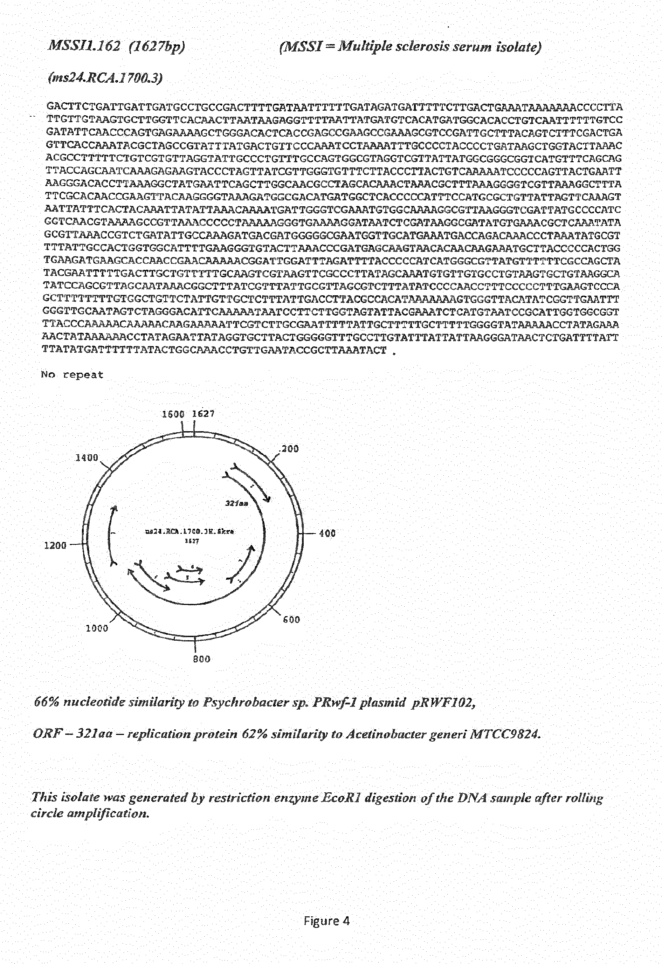

Abbreviations for Figures 1-4: Rep = replication protein; CP = capsid protein
CMI:cattle milk isolate
HCBI: healty cattle blood isolate
MSCI: MS brain isolate
MSSI: MS serum isolate
Sphinx: slow progressive hidden infection of variable (X) latency

### Figure 1:

### Group 1 - Isolates from bovine serum, bovine milk and multiple sclerosis (MS) brain samples (post mortem) - all related to Sphinx1.76

(a) HCBI6.252 (2522 bp) (Healthy Cattle Blood Isolate; identical to CMI1.252 (Cow Milk Isolate)).
(b) HCBI6.159 (1591 bp): A deletion in HCBI6.252 apparently resulted in HCBI6.159 - both were rescued independently from the bovine serum using back-to-back primer, i.e. circular DNA molecules and not an artefact. This could indicate that these infectious organisms have 2 forms - a large and small molecule related to or complementing each other
(c) MSBI1.176 (Multiple Sclerosis Brain Isolate) (1766 bp) - 98% similar to Sphinx1.76
(d) MSBI2.176 (1766 bp) - isolated from the same MS-brain sample as MSB1.176
(e) CMI1.252 (2523 bp) (Cow Milk Isolate; identical to HCBI6.252)
(f) CMI2.214 (2148 bp)
(g) CMI3.168 (1687 bp)
(h) CMI4.158 (1583 bp)

The isolates were all generated by using back-to-back primers designed on the replication gene of Sphinx1.76.
Primers (several isolates were isolated twice by applying both primer pairs independently).
Nn (forward GGATTAATGCCAATGATCC), Xn (reverse CTTTGCCTGTTTCTCT CG), and/or No (forward GAGGACGAATTAATATTACAAGTC), Xo (reverse GTTCTCGTTTTCTTGGTAA)

### Figure 2:

### Group 2: Isolate HCBI7.228(2280bp) (Healthy Cattle Blood Isolate) related to Sphinx2.36

This sample was generated by PCR from bovine serum using back-to-back primers
Primers: Nd (forward CAGATTGCAAAGCCTGTAATTCAT), Xd (reverse CTAAGGCAGATCAACACAGGGATA)

### Figure 3:

### Group 3: Isolates from bovine serum and multiple sclerosis serum and MS brain samples (post mortem) - distantly related to known ssDNA viruses

Nucleotide sequences of (a) HCBI8.215 (2152 bp) and (b) HCBI9.212 (2121 bp) (Healthy Cattle Blood Isolate) as well as (c) MSSI2.225 (2259 bp) (Multiple Sclerosis Serum Isolate). An identical sequence (MSBI3.224) to MSSI2.225 was also isolated from an MS-brain sample.
These HCBI isolates were generated as follows:
Serum samples were subjected to ultracentrifugation on Optiprep gradient centrifugation. The gradient was fractionated, DNA extracted from fractions and subjected to rolling circle amplification. Products were digested with BamHI or EcoRI enzymes. Products were cloned and sequenced. Complete genomes were obtained by amplification of original DNA samples with back-to-back primers designed on the respective gradient clones. Complete genomes were sequenced in full.

The MSSI isolate (as well as the clone MSB13.224 from the MS-brain) was obtained after rolling circle amplification of the extracted DNA from MS serum and MS brain, restriction enzyme digestion, cloning of the product and subsequent sequencing. The complete genome was obtained by amplification with back-to-back primers designed on the original clone. The complete genome was sequenced in full.

### Figure 4:

### Group 4 - distantly related to Psychrobacter spp plasmid: Nucleotide sequence of MSSI1.162 and putative open reading frames

MS-serum isolate MSSI1.162 (group 4) was generated by rolling circle amplification of a multiple MS-serum sample. The product was digested with restriction enzyme HindIII, cloned and sequenced.

### Figure 5:

### Genome organisation of 4 groups of isolates

The isolates are grouped according to their sequence similarity to the Sphinx1.76 genome (group 1), Sphinx2.36 (group 2), myco-like Gemycircularviruses (group 3) and Psychrobacter spp. Plasmid (group 4).
(A) group 1
(B) groups 2,3 and 4

Figure 6:
   Alignment of a replication gene/ iteron-like repeat region between 8 isolates and Sphinx1.76
Figure 7:
   Schematic outline of latent infection of different types of brain cells with Herpes type genomes and BMF factor
Figure 8:
   Spontaneous reactivation of Herpes DNA in a cell concomitantly infected by Herpes and BMF DNA
   Amplification of BMF and inhibition of Herpes DNA replication.
Figure 9:
   Schematic outline of the recognition of foreign antigens by the immune system, T cell response
Figure 10:
   Mononuclear inflammatory cells surrounding a small vein in an early lesion
   Lymphocytes, monocytes, plasma cells and occasional macrophages.
Figure 11:
   Advancing age of the lesion (plaque) on the left with normal white matter on the right
   Macrophages are present in the lesion (arrows) and at the interphase.
Figure 12:
   Schematic outline of the pathogenesis concept for multiple sclerosis
   EBV is used as an example for the role of Herpes-type viruses.
Figure 13:
   Tentative Scheme of MS pathogenesis

### Detailed description of the present invention

In the present application a new concept for the pathogenesis of multiple sclerosis is presented: Interaction of an Amplifying Virus and the amplified DNA of a Bovine Milk (or serum) Factor (BMF)

### Introduction

The incidence of multiple sclerosis (MS) increased in several parts of the world (*reviewed in Kurtzke, 2000, Alcalde-Cabero et al., 2013*). This increase has been mainly attributed to environmental factors. Migrants from high to lower risk areas retain the MS risk of their birth place only if they are at least age 15 at migration, frequently interpreted to be due to an infection acquired during early childhood (*reviewed in Kurtzke, 2000*). Clustering of cases and the geographic epidemiology has also been widely discussed: A rising incidence of MS was noted in females linked to urbanization (*Kotzamani et al., 2012).*

Demyelinization is a characteristic feature of MS lesions. Four fundamentally different patterns of demyelination were found, defined on the basis of myelin protein loss, the location and extension of plaques, the patterns of oligodendrocyte destruction, and the immunopathological evidence of complement activation (*Lucchinetti et al., 2000, Metz et al., 2014*)*.* At a given time point of the disease the patterns of demyelination were heterogeneous between patients; but they were homogenous within multiple active lesions from the same patient, potentially pointing to different contributing factors.

Two of the risk factors seem to deserve special attention: the relatively consistent results pointing to a possible role of different, predominantly herpes-group viruses, and the consumption of fresh cow milk, potentially including other dairy products (*see below*). In addition, Vitamin D deficiency plays a role as a risk factor.

### - Viral Risk Factors

Apparently all Herpes virus types share two properties which seem to be relevant for the subsequent discussion:
During their persistence in a latent stage, spontaneous reactivation may occur, in part regulated by specific gene functions, partly also by epigenetic mechanisms (*reviewed in Nicoll et al., 2012, Grinde, 2013*). Reactivation may also be triggered by interaction with extracellular cytokines, such as transforming growth factor β.

Spontaneous induction of a lytic cycle has been observed for virtually every human pathogenic Herpes virus type. The high antibody titers against Epstein-Barr virus structural proteins in EBV-positive Burkitt's lymphomas and nasopharyngeal cancers (*reviewed in Henle, W. and Henle, G.,* 1977) may serve here as one example. Reactivations of human Herpes virus type 6, Varicella-Zoster virus, Herpes simplex virus and others are not rare events and may affect a number of different cell types (*Hu Knox et al., 2000).*

A second remarkable property of herpes virus infection represents the amplification of various double- or single-stranded small DNA virus genomes upon infection of cells containing such DNAs in a latent state. This has been noted for human and monkey Polyoma viruses, JC and SV40, for human and bovine Papilloma viruses, as well as for single-stranded Adeno-associated (AAV) and Anello-/TT-viruses (*Schlehofer and zur Hausen, 1990, Heilbronn et al., 1993, Borkosky et al., 2012).* The Herpes-group viruses used in these studies were Herpes simplex virus, human cytomegalovirus and Epstein-Barr virus. The potential to induce amplification of latent small viral DNA genomes is also shared by Adeno- and Vaccinia viruses (*Schlehofer and zur Hausen,* 1990). The helper effect of Herpes- and adenovirus-induced amplification of parvoviruses has been intensively studied for adeno-associated viruses. The replication of the latter seems to depend on this helper effect but in turn leads to a reduction of Herpes- or adenovirus replication due to the preferential amplification of small viral DNA (*Schlehofer et al., 1983, Matz* et *al., 1984, Bantel-Schaal and zur Hausen, 1988, Schmitt et al., 1989, Schlehofer and zur Hausen, 1990, Heilbronn et al., 1990a, Heilbronn et al., 1990b).*

Spontaneous induction of Herpes-group viruses and the amplification of latent small viral DNA form the basis for the subsequent postulation of the mechanism underlying MS development.

### - Bovine milk factor

Several reports noted a correlation between consumption of non-pasteurized cow milk and MS development (*Murray TJ. 1976, Sepcić et al., 1993, Malosse and Perron, 1993),* whereas others stressed long-time consumption of cow milk as a risk factor, in particular when consumed in the early phase of life (*Agranoff and Goldberg, 1974, Christensen, 1975, Warren, 1984, Butcher, 1976, 1986, Winer et al., 2001, Munger et al., 2011a).*

If a specific factor in cow milk exists which increases the risk for MS development, one can anticipate a protective role of long-term breast-feeding. Long-term breast-feeding (for six months and more) has indeed repeatedly been reported as having a protective effect for MS development (*Christensen, 1975, Warren, 1984, Tarrats et al., 2002, Conradi et al., 2013*). The existence of a cow milk factor would also not exclude a specific genetic predisposition for the development of MS. A monogenic predisposition for MS has been reported in a chromosomal localization close to BRCAI (*Holzmann et al., 2013)* .

### - Vitamin-D deficiency

A role of vitamin-D deficiency has repeatedly been implicated for the initiation of MS (*reviewed in Ascherio et al. 2012, 2013*).

A convincing relationship between vitamin D deficiency and Epstein Barr Virus reactivation originates from early studies on EBV reactivation by transforming growth factor beta (TGF-β). A serum factor, purified and labeled as Epstein-Barr virus-inducing factor (EIF) (*Bauer et al., 1982*) proved to be identical to the subsequently described TGF-β molecule *(Frolik et al., 1983, Bauer et al., 1991).* TGF-β in turn is negatively regulated by activated vitamin D receptors *(Isik et al., 2012, Ito* et *al., 2013, Zerr et al., 2014).* This could very well explain the season-related preferential onset of MS and of exacerbations.
The relationship between low vitamin D and EBV reactivation is further supported by studies describing a correlation between low vitamin D and elevated immunoreactivity against Epstein-Barr virus prior to the clinical manifestation of multiple sclerosis (*Munger et al., 2011b, Décard et al., 2012*) and a higher rate of EBV excretion of EBV-positive MS patients in comparison to EBV-positive healthy controls (*Yea et al., 2013*).

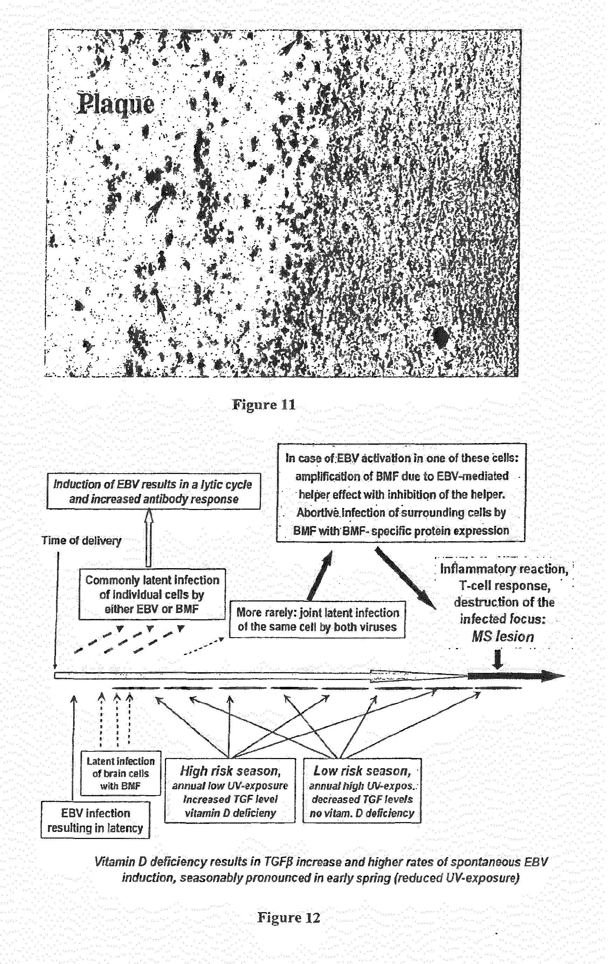

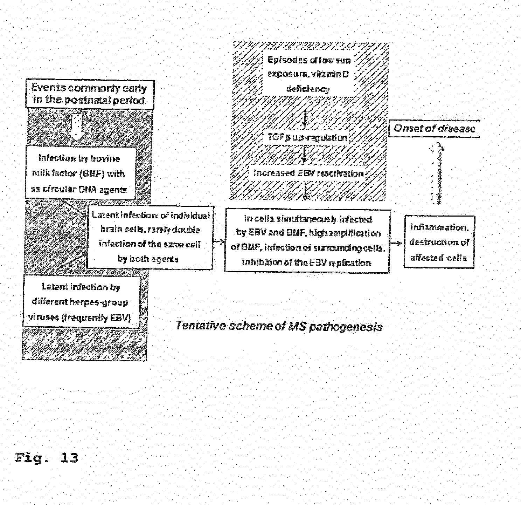

Thus, at least two factors have been implicated as potential etiological contributors for both diseases of the CNS (e.g. MS) and cancer (e.g. colon and breast cancer): vitamin D deficiency and the reactivation of various herpes group viruses, mainly Epstein-Barr virus (EBV), human herpes virus type 6, and varicella-zoster virus. According to the present invention the identification of several novel types of small circular single-stranded DNAs, presumably of viral origin, from cattle sera of the present invention and commercially available dairy products, show a unifying concept. The inventors have demonstrated in the present invention that co-infection of cells with herpes-group viruses and small single-stranded or double-stranded DNA viruses results in an substantial amplification of small viral DNA with partial inhibition of the herpes virus. Some of the molecules identified in dairy cattle sera and milk are distantly related to DNA reported in prion-linked brain lesions and have been found in two autopsy lesions of patients with multiple sclerosis. The amplification of these single-stranded DNA molecules by reactivation of a co-latently persisting herpes virus genome could result in their amplification and evoking a local immune response resulting in destruction of the affected brain cells. This model could in part explain the North-South incidence gradient of multiple sclerosis, which is thought to be linked to vitamin-D deficiency and herpes virus reactivation (c.f. Fig. 12 and 13).

In addition, the full-length genomes of the isolates from MS brains and sera were isolated and re-circularized before transfection into the human cell line 293TT. Transfected cells were harvested on day 3 and total RNA extracted using the miRNA Easy Kit (Qiagen). Samples were further purified (DNase digestion, ribosome removal) and subjected to high throughput RNA sequencing. RNA transcripts have been obtained for MSBI1,176, MSBI2,176 (Group 1), MSSI2.225 (Group 3) and MSSI1.162 (Group 4). The RNA transcripts clearly show that the isolates replicate in human cells.

The inventors consider Vitamin D deficiency and herpes virus reactivations as risk factors also for breast and colon cancers. Reactivation of dual latent infections within the same cell, outlined above for multiple sclerosis pathogenesis, could therefore also play a particular important role in the aetiology of these cancers.

Thus, it is considered by the inventors that multiple sclerosis (MS) and also the other below mentioned diseases result from
- Latent infections of the same cell with two different infectious agents, one of them most likely a herpes-type virus (in particular EBV, HHV-6, VZV, but also HSV, HHV-7), the other one acquired by bovine milk consumption (bovine milk factor - BMF) as the first event. Each of them latently infects individual cells, but occasionally genomes of both agents occur within the same cell.
- Reactivation of the herpes-type virus most frequently, but not only, Epstein Barr virus (EBV) to a lytic cycle as a second precondition. For EBV this is probably linked to increased levels of transforming growth factor β (TGF β) which is negatively regulated by activated vitamin D receptors;
- As third event, amplification of BMF, resulting in partial suppression of Herpes-type DNA synthesis and formation of BMF particles or spreading of its nucleic acid to neighbouring cells via neuronal interconnections;
- This is followed by an infection of neighbouring cells with expression of BMF protein;
- Finally, T-cell response against BMF leads to the destruction of affected cells and in case of MS to plaque formation. This supports the clinical observation of the focal appearance of lesions, commonly starting from a central vein and the intensive localized immune response in early lesions.

Transmissions of agents present in milk or dairy products may lead to latent infections in human brain cells followed by amplification of these agents in case of co-latency and spontaneous induction of a Herpes virus DNA or Herpes virus - like DNA. Potential BMF candidate agents are described in Examples 2-5 and the accompanying figures.

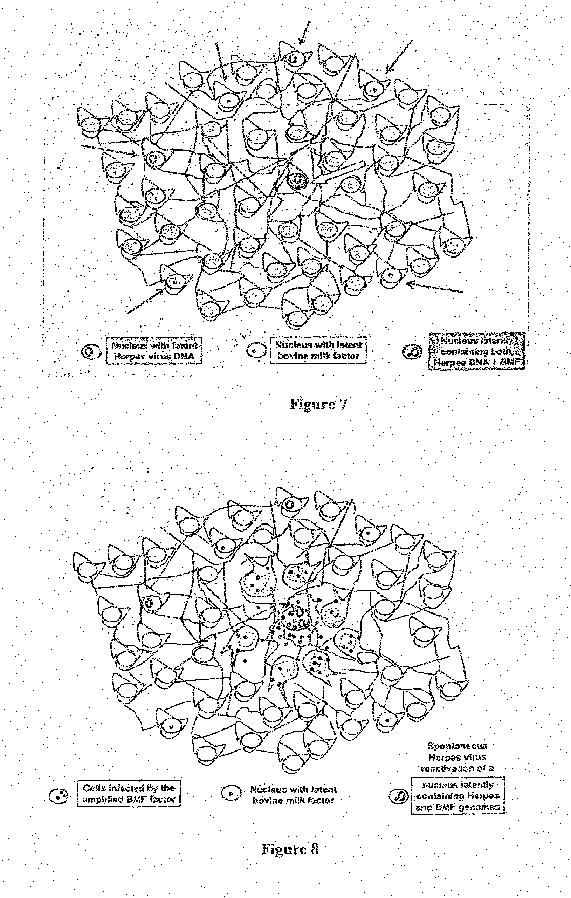

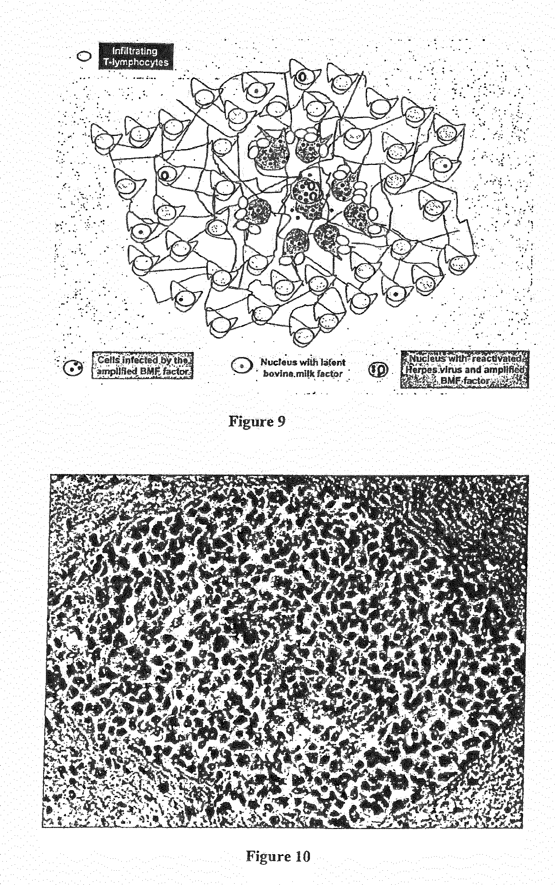

The presence of presumably circular single stranded DNA related to Sphinx-sequences, Anello-, Circo-, and Gemycircularvirus families, as well as *Psychrobacter* species in cattle sera (see Examples 2-5), in commercially available milk samples, as well as in florid MS lesions and MS serum permits the development of a concept for MS pathogenesis. It integrates observations of involvement of Herpes-type viruses, most prominently of EBV, of their property to amplify small double- and single-stranded DNA viruses, of viral cow milk factors, of vitamin-D deficiency, the EBV inducing property of TGFβ, and the partial season dependence of MS onset and of exacerbation in the course of disease. This concept is schematically outlined in Figures 7-13.

An initial dual latent infection of the same or closely flanking cells by a herpes virus genome and the postulated BMF, followed by a trigger for Herpes virus reactivation and the subsequent preferential amplification of single-stranded DNA are defined as the primary event. In the case of latent EBV infection, vitamin D deficiency with the subsequent up-regulation of TNF-β as an EBV-inducing factor could be the important trigger for up-regulation. Probably abortive infection of neighbouring cells with viral antigen expression results in an active T-cell response and the destruction of the affected cells. The frequently described seasonality of MS onset and of new rounds of MS exacerbations, the repeatedly reported North-South gradient of MS incidence should reflect the degree of sun-light exposure, negatively correlating vitamin D levels with TGFβ concentration and EBV reactivation.

Thus, the inventors anticipate the presence of different BMF sequences also in susceptible normal human brain cells in a latent form. The remarkable heterogeneity of the BMF isolates may also find its reflection in variations for pathologic characteristics of MS in humans (*Lucchinetti et al., 2000, Metz et al., 2014*). It would not be too surprising if eventually "high" and "low" risk types will be identified, in a certain analogy to human papillomavirus pathogenicity (*zur Hausen, 1985*). The majority of those carriers will not develop MS, since the latter should require latent co-infection of a BMF-positive cell with a Herpes-type virus and spontaneous induction of the latter. This should be a rare event, increasing, however, under conditions resulting in frequent Herpes virus reactivations.

As a final point, it should be of interest to also apply this concept to other presumably autoimmune diseases and certain cancers occurring at increased frequency under conditions of vitamin D deficiency. As far as cancers are concerned, this specifically accounts for colon- and breast cancer, and possibly for ovarian, prostate, pancreatic cancer and lung cancers.

The risk for insulin-dependent diabetes mellitus has been repeatedly linked to cow milk consumption and vitamin D deficiency (*reviewed in Scott, 1990, in Grant, 2006, in Hyppönen et al, 2010*). The latter system seems to come particularly close to the MS situation.

Accordingly, the present invention relates to MSBI1.176 or MSBI2.176 polynucleic acid comprising:
(a) a nucleotide sequence depicted in any one of Figure 1 (c) or 1(d);
(b) a nucleotide sequence being complementary to a nucleotide sequence of (a);
   (
(c) a nucleotide sequence which is redundant as a result of the degeneracy of the genetic code compared to any of the above-given nucleotide sequences.

The term "polynucleic acid" refers to a single-stranded or double-stranded nucleic acid sequence. A polynucleic acid may consist of deoxyribonucleotides or ribonucleotides, nucleotide analogues or modified nucleotides or may have been adapted for diagnostic or therapeutic purposes. A polynucleic acid may also comprise a double stranded cDNA clone which can be used, for example, for cloning purposes.

The MSBI1.176 or MSBI2.176 polynucleic acids of the invention can be prepared according to well-known routine methods, for example, by (a) isolating the entire DNA or RNA from a sample, (b) detecting the MSBI1.176 or MSBI2.176 sequence by hybridization or PCR and (c) cloning of the MSBI1.176 or MSBI2.176 sequence into a vector.

Also included within the present invention are sequence variants of the polynucleic acid of the invention containing either deletions and/or insertions of one or more nucleotides, especially insertions or deletions of one or more codons, mainly at the extremities of oligonucleotides (either 3' or 5') and which show at least 90%, 91%, 92%, 93%, 94%, 95%, 96%, 97%, 98% or 99% identity to said polynucleic acid sequences of the invention. Polynucleic acid sequences according to the present invention which are similar to the sequences as shown in Figure 1(c) or 1(d) can be characterized and isolated according to any of the techniques known in the art, such as amplification by means of sequence-specific primers, hybridization with sequence-specific probes under more or less stringent conditions, sequence determination of the genetic information of MSBI1.176 or MSBI2.176 etc.

The present invention also provides fragments of the nucleotide sequences of the present invention described above that harbour a replication gene which codes for a replication protein. An autonomous replicating nucleotide sequence comprises a nucleotide sequence of the replication gene or a fragment thereof which is capable of inducing autonomous replication. Replication protein represents an endonuclease which binds single-stranded DNA inducing a single-stranded cut at or near the origin of replication (Wolds, 1997). The skilled person can derive at such fragments capable of inducing autonomous replication without undue experimentation. Such fragments may have a length of at least 45, at least 55, or at least 65 nt.

The person skilled in the art can easily determine which nucleic acid sequences are related to a nucleotide sequence of Figure 1(c) or 1(d) to 4 or which fragments are still capable of replicating autonomously by using standard assays.

The present invention also provides polynucleic acid sequences which are redundant as a result of the degeneracy of the genetic code compared to any of the above-given nucleotide sequences. These variant polynucleic acid sequences will thus encode the same amino acid sequence as the polynucleic acids they are derived from.

The MSBI1.176 or MSBI2.17polynucleic acids of the invention might be present as an extrachromosomal episome, might be integrated into the host's genome and/or might be linked to a host cell DNA.

The present disclosure also relates to an oligonucleotide primer comprising or consisting of part of a polynucleic acid as defined above, with said primer being able to act as primer for specifically sequencing or specifically amplifying MSBI1.176 or MSBI2.176 polynucleic acid of the invention.

The term "primer" disclosed herein refers to a single stranded DNA oligonucleotide sequence capable of acting as a point of initiation for synthesis of a primer extension product which is complementary to the nucleic acid strand to be copied. The length and the sequence of the primer must be such that they allow priming the synthesis of the extension products. Preferably the primer is about 5-50 nucleotides. Specific length and sequence will depend on the complexity of the required DNA or RNA targets, as well as on the conditions of primer use such as temperature and ionic strength.

The fact that amplification primers do not have to match exactly with a corresponding template sequence to warrant proper amplification is amply documented in the Literature. The amplification method used can be, for example, polymerase chain reaction (PCR), ligase chain reaction (LCR), nucleic acid sequence-based amplification (NASBA), transcription-based amplification system (TAS), strand displacement amplification (SDA) or amplification by means of Qb replicase or any other suitable method to amplify nucleic acid molecules using primer extension. During amplification, the amplified products can be labelled either using labelled primers or by incorporating labelled nucleotides.

Labels may be isotopic (32P, 35S, etc.) or non-isotopic (biotin, digoxigenin, etc.). The amplification reaction is repeated between 20 and 70 times, advantageously between 25 and 45 times.

Any of a variety of sequencing reactions known in the art can be used to directly sequence the viral genetic information and determine the ORF by translating the sequence of the sample into the corresponding amino acid sequence. Exemplary sequencing reactions include those based on techniques developed by Sanger or Maxam and Gilbert. It is also contemplated that a variety of automated sequencing procedures may be utilized when performing the subject assays including sequencing by mass spectrometry (see, for example: PCT publication WO 94/16101). It will be evident to one skilled in the art that, for example the occurrence of only two or three nucleic bases needs to be determined in the sequencing reaction.

Preferably, these primers disclosed herein are about 5 to 50 nucleotides long, more preferably from about 10 to 25 nucleotides. Most preferred are primers disclosed herein having a length of at least 13 bases.

The present invention also relates to an oligonucleotide probe comprising or consisting of part of an MSBI1.176 or MSBI2.176 polynucleic acid as defined above, with said probe being able to act as a hybridization probe for specific detection of a MSBI1.176 or MSBI2.176 polynucleic acid according to the invention.

The probe can be labelled or attached to a solid support.

The term "probe" refers to single stranded sequence-specific oligonucleotides which have a sequence which is complementary to the target sequence of an MSBI1.176 or MSBI2.176 polynucleic acid to be detected.

Preferably, these probes are about 5 to 50 nucleotides long, more preferably from about 10 to 25 nucleotides. Most preferred are probes having a length of at least 13 bases.

The term "solid support" can refer to any substrate to which an oligonucleotide probe can be coupled, provided that it retains its hybridization characteristics and provided that the background level of hybridization remains low. Usually the solid substrate will be a microtiter plate, a membrane (e.g. nylon or nitrocellulose) or a microsphere (bead). Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin or haptens.

The oligonucleotides according to the present disclosure, used as primers or probes may also contain or consist of nucleotide analogues such as phosphorothioates, alkylphosphoriates or peptide nucleic acids or may contain intercalating agents. These modifications will necessitate adaptions with respect to the conditions under which the oligonucleotide should be used to obtain the required specificity and sensitivity. However, the eventual results will be essentially the same as those obtained with the unmodified oligonucleotides.

The introduction of these modifications may be advantageous in order to positively influence characteristics such as hybridization kinetics, reversibility of the hybrid-formation, biological stability of the oligonucleotide molecules, etc.

The polynucleic acids of the invention may be comprised in a composition of any kind. Said composition disclosed herein may be for diagnostic, therapeutic or prophylactic use.

The present invention also relates to a recombinant expression vector comprising an MSBI1.176 or MSBI2.176 polynucleic acid of the invention as defined above operably linked to prokaryotic, eukaryotic or viral transcription and translation control elements as well as host cells containing such vector.

The term "vector" may comprise a plasmid, a cosmid, an artificial chromosome, a phage, or a virus or a transgenic non-human animal. Particularly useful for vaccine development may be MSBI1.176 or MSBI2.176 recombinant molecules, BCG or adenoviral vectors, as well as avipox recombinant viruses.

The term "recombinant expression" used within the context of the present invention refers to the fact that the polypeptides of the present invention are produced by recombinant expression methods be it in prokaryotes, or lower or higher eukaryotes as discussed in detail below.

The term "host cell" refers to cells which can be or have been, used as recipients for a recombinant vector or other transfer polynucleotide, and include the progeny of the original cell which has been transfected.

It is understood that the progeny of a single parental cell may not necessarily be completely identical in morphology or in genomic or total DNA complement as the original parent, due to natural, accidental, or deliberate mutation or recombination.

The term "lower eukaryote" refers to host cells such as yeast, fungi and the like. Lower eukaryotes are generally (but not necessarily) unicellular. Preferred lower eukaryotes are yeasts, particularly species within Saccharomyces, Schizosaccharomyces, Kluiveromyces, Pichia (e. g. Pichia pastoris), Hansenula (e. g. Hansenula polymorph), Schwaniomyces, Schizosaccharomyces, Yarowia, Zygosaccharomyces and the like. Saccharomyces cerevisiae, S. carlsbergensis and K. lactis are the most commonly used yeast hosts, and are convenient fungal hosts.

The term "higher eukaryote" refers to host cells derived from higher animals, such as mammals, reptiles, insects, and the like. Presently preferred higher eukaryote host cells are derived from Chinese hamster (e. g. CHO), monkey (e. g. COS and Vero cells), baby hamster kidney (BHK), pig kidney (PK15), rabbit kidney 13 cells (RK13), the human osteosarcoma cell line 143 B, the human cell line HeLa and human hepatoma cell lines like Hep G2, the 293TT cell line (Buck et al., 2004) and insectcell lines (e.g. Spodoptera frugiperda). The host cells may be provided in suspension or flask cultures, tissue cultures, organ cultures and the like. Alternatively the host cells may also be transgenic non-human animals.

The term "prokaryotes" refers to hosts such as E. coli, Lactobacillus, Lactococcus, Salmonella, Streptococcus, Bacillus subtilis or Streptomyces. Also these hosts are contemplated within the present invention.

The segment of the MSBI1.176 or MSBI2.176 DNA encoding the desired sequence inserted into the vector sequence may be attached to a signal sequence. Said signal sequence may be that from a non-MSBI1.176 or MSBI2.176 source, but particularly preferred constructs according to the present invention contain signal sequences appearing in the MSBI1.176 or MSBI2.176 genome before the respective start points of the proteins.

Higher eukaryotes may be transformed with vectors, or may be infected with a recombinant virus, for example a recombinant vaccinia virus. Techniques and vectors for the insertion of foreign DNA into vaccinia virus are well known in the art, and utilize, for example homologous recombination. A wide variety of viral promoter sequences, possibly terminator sequences and poly(A)-addition sequences, possibly enhancer sequences and possibly amplification sequences, all required for the mammalian expression, are available in the art. Vaccinia is particularly preferred since vaccinia halts the expression of host cell proteins. For vaccination of humans the avipox and Ankara Modified Virus (AMV) are particularly useful vectors.

Also known are insect expression transfer vectors derived from baculovirus Autographa californica nuclear polyhedrosis virus (AcNPV), which is a helper-independent viral expression vector. Expression vectors derived from this system usually use the strong viral polyhedrin gene promoter to drive the expression of heterologous genes. Different vectors as well as methods for the introduction of heterologous DNA into the desired site of baculovirus are available to the person skilled in the art for baculovirus expression. Also different signals for posttranslational modification recognized by insect cells are known in the art.

The present invention also relates to a polypeptide having an amino acid sequence encoded by an MSBI1.176 or MSBI2.176 polynucleic acid as defined above, or a part or an analogue thereof being substantially similar and biologically equivalent.

The term "polypeptide" refers to a polymer of amino acids and does not refer to a specific length of the product. Thus, peptides, oligopeptides, and proteins are included within the definition of polypeptide. This term also does not refer to or exclude post-expression modifications of the polypeptide, for example, glycosylations, acetylations, phosphorylations and the like. Included within the definition are, for example, polypeptides containing one or more analogues of an amino acid (including, for example, unnatural amino acids, peptide nucleic acid (PNA), etc.), polypeptides with substituted linkages, as well as other modifications known in the art, both naturally occurring and non-naturally occurring.

The polypeptides according to the present invention contain preferably at least 3, preferably 4 or 5 contiguous MSBI1.176 or MSBI2.176 amino acids, 6 or 7 preferably however at least 8 contiguous MSBI1.176 or MSBI2.176 amino acids, at least 10 or at least 15.

The polypeptides of the invention, and particularly the fragments, can be prepared by classical chemical synthesis. The synthesis can be carried out in homogeneous solution or in solid phase. The polypeptides according to this invention can also be prepared by means of recombinant DNA techniques. The present invention also relates to a method for production of a recombinant polypeptide as defined above, comprising: (a) transformation of an appropriate cellular host with a recombinant vector, in which a polynucleic acid or a part thereof as defined above has been inserted under the control of the appropriate regulatory elements, (b) culturing said transformed cellular host under conditions enabling the expression of said insert, and (c) harvesting said polypeptide.

The present invention also relates to an antibody raised upon immunization with at least one polypeptide as defined above, with said antibody being specifically reactive with any of said polypeptides, and with said antibody being preferably a monoclonal antibody. The term "antibody", preferably, relates to antibodies which consist essentially of pooled monoclonal antibodies with different epitopic specificities, as well as distinct monoclonal antibody preparations. Monoclonal antibodies are made from an antigen containing, e.g., a polypeptide encoded by an MSBI1.176 or MSBI2.176 polynucleic acid of the invention or a fragment thereof by methods well known to those skilled in the art. As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. Thus, these fragments are preferred, as well as the products of a FAB or other immunoglobulin expression library. Moreover, antibodies useful for the purposes of the present invention include chimerical, single chain, and humanized antibodies.

The present invention also relates to diagnostic and therapeutic approaches using cell-mediated immune responses.

Preferably, the antibody or antigen binding fragment thereof carries a detectable label. The antibody/fragment can be directly or indirectly detectably labeled, for example, with a radioisotope, a fluorescent compound, a bioluminescent compound, a chemiluminescent compound, a metal chelator or an enzyme. Those of ordinary skill in the art will know of other suitable labels for binding to the antibody, or will be able to ascertain such, using routine experimentation.

The present disclosure also relates to a diagnostic kit for use in determining the presence of an HCBI, MSBI or CMI polynucleic acid or polypeptide of the invention, said kit comprising a primer, a probe, and/or an antibody of the invention. Said kit may have any format well known to the person skilled in the art, e.g. can be an ELISA-based kit.

The present invention also relates to a method for the detection of an MSBI1.176 or MSBI2.176polynucleic acid according to the invention present in a biological sample, comprising: (a) optionally extracting sample polynucleic acid, (b) amplifying the polynucleic acid as described above with at least one primer as disclosed above, optionally a labelled primer, and (c) detecting the amplified polynucleic acids.

The term "polynucleic acid" can also be referred to as analyte strand and corresponds to a single- or double-stranded polynucleic acid molecule.

The term "labelled" refers to the use of labelled nucleic acids. This may include the use of labelled nucleotides incorporated during the polymerase step of the amplification or labelled primers, or by any other method known to the person skilled in the art.

The present invention also relates to a method for the detection of an MSBI1.176 or MSBI2.176polynucleic acid according to the invention present in a biological sample, comprising: (a) optionally extracting sample polynucleic acid, (b) hybridizing the polynucleic acid as described above with at least one probe as defined above, and (c) detecting the hybridized polynucleic acids.

The hybridization and washing conditions are to be understood as stringent and are generally known in the art. However, according to the hybridization solution (SSC, SSPE, etc.), these probes should be hybridized at their appropriate temperature in order to attain sufficient specificity.

According to the hybridization solution (SSC, SSPE, etc.), these probes should be stringently hybridized at their appropriate temperature in order to attain sufficient specificity. However, by slightly modifying the DNA probes, either by adding or deleting one or a few nucleotides at their extremities (either 3' or 5'), or substituting some non-essential nucleotides (i. e. nucleotides not essential to discriminate between types) by others (including modified nucleotides or inosine) these probes or variants thereof can be caused to hybridize specifically at the same hybridization conditions (i.e. the same temperature and the same hybridization solution). Also changing the amount (concentration) of probe used may be beneficial to obtain more specific hybridization results. It should be noted in this context, that probes of the same length, regardless of their GC content, will hybridize specifically at approximately the same temperature in TMACI solutions.

Suitable assay methods for purposes of the present invention to detect hybrids formed between the oligonucleotide probes and the MSBI1.176 or MSBI2.176 polynucleic acid sequences in a sample may comprise any of the assay formats known in the art, such as the conventional dot-blot format, sandwich hybridization or reverse hybridization. For example, the detection can be accomplished using a dot blot format, the unlabelled amplified sample being bound to a membrane, the membrane being incorporated with at least one labelled probe under suitable hybridization and wash conditions, and the presence of bound probe being monitored.

An alternative and preferred method is a "reverse" dot-blot format, in which the amplified sequence contains a label. In this format, the unlabelled oligonucleotide probes are bound to a solid support and exposed to the labelled sample under appropriate stringent hybridization and subsequent washing conditions. It is to be understood that also any other assay method which relies on the formation of a hybrid between the polynucleic acids of the sample and the oligonucleotide probes according to the present invention may be used.

The present invention also relates to a method for detecting a polypeptide encoded by an MSBI1.176 or MSBI2.176polynucleic acid of the present invention or an antibody against said polypeptide present in a biological sample, comprising: (a) contacting the biological sample for the presence of such polypeptide or antibody as defined above, and (b) detecting the immunological complex formed between said antibody and said polypeptide.

The immunoassay methods according to the present invention may utilize antigens from different domains of the new and unique polypeptide sequences of the present invention. It is within the scope of the invention to use for instance single or specific oligomeric antigens, dimeric antigens, as well as combinations of single or specific oligomeric antigens. The MSBI1.176 or MSBI2.176 antigens of the present invention may be employed in virtually any assay format that employs a known antigen to detect antibodies or cell-mediated immune responses. Thus, the present invention also encompasses the detection of cell-mediated immune responses against MSBI1.176 or MSB12.176 antigens and the application of therapeutic interferences based on cell-mediated immune responses against MSBI1.176 or MSBI2.176 antigens.

Of course, an assay format that denatures the MSBI1.176 or MSBI2.176 conformational epitope should be avoided or adapted. A common feature of all of these assays is that the antigen is contacted with the body component suspected of containing HCBI, MSBI, MSSI or CMI antibodies under conditions that permit the antigen to bind to any such antibody present in the component. Such conditions will typically be physiologic temperature, pH and ionic strength using an excess of antigen. The incubation of the antigen with the specimen is followed by detection of immune complexes comprised of the antigen.

Design of the immunoassays is subject to a great deal of variation, and many formats are known in the art. Protocols may, for example, use solid supports, or immunoprecipitation. Most assays involve the use of labeled antibody or polypeptide; the labels may be, for example, enzymatic, fluorescent, chemiluminescent, radioactive, or dye molecules. Assays which amplify the signals from the immune complex are also known; examples of which are assays which utilize biotin and avidin or streptavidin, and enzyme-labeled and mediated immunoassays, such as ELISA assays.

The immunoassay may be in a heterogeneous or in a homogeneous format, and of a standard or competitive type. In a heterogeneous format, the polypeptide is typically bound to a solid matrix or support to facilitate separation of the sample from the polypeptide after incubation. Examples of solid supports that can be used are nitrocellulose (e. g., in membrane or microtiter well form), polyvinyl chloride (e. g., in sheets or microtiter wells), polystyrene latex (e. g., in beads or microtiter plates, polyvinylidine fluoride (known as Immunolon), diazotized paper, nylon membranes, activated beads, and Protein A beads. The solid support containing the antigenic polypeptides is typically washed after separating it from the test sample, and prior to detection of bound antibodies. Both standard and competitive formats are known in the art.

In a homogeneous format, the test sample is incubated with the combination of antigens in solution. For example, it may be under conditions that will precipitate any antigen-antibody complexes which are formed. Both standard and competitive formats for these assays are known in the art.

In a standard format, the amount of MSBI1.176 or MSBI2.176 antibodies in the antibody-antigen complexes is directly monitored. This may be accomplished by determining whether (labelled) anti-xenogeneic (e. g. anti-human) antibodies which recognize an epitope on anti-MSBI1.176 or anti-MSBl2.176odies will bind due to complex formation. In a competitive format, the amount of MSBI1.176 or MSBI2.176 antibodies in the sample is deduced by monitoring the competitive effect on the binding of a known amount of labelled antibody (or other competing ligand) in the complex.

Complexes formed comprising anti-MSBI1.176 or anti-MSBI2.176 antibody (or in the case of competitive assays, the amount of competing antibody) are detected by any of a number of known techniques, depending on the format. For example, unlabeled MSBI1.176 or MSBI2.17 antibodies in the complex may be detected using a conjugate of anti-xenogeneic Ig complexed with a label (e. g. an enzyme label).

In an immunoprecipitation or agglutination assay format the reaction between the MSBI1.176 or MSBI2. 176 antigens and the antibody forms a network that precipitates from the solution or suspension and forms a visible layer or film of precipitate. If no anti-MSBI1.176 or anti-MSBI2.176antibody is present in the test specimen, no visible precipitate is formed.

There currently exist three specific types of particle agglutination (PA) assays. These assays are used for the detection of antibodies to various antigens when coated to a support. One type of this assay is the hemagglutination assay using red blood cells (RBCs) that are sensitized by passively adsorbing antigen (or antibody) to the RBC. The addition of specific antigen/antibodies present in the body component, if any, causes the RBCs coated with the purified antigen to agglutinate.

To eliminate potential non-specific reactions in the hemagglutination assay, two artificial carriers may be used instead of RBC in the PA. The most common of these are latex particles.

The solid phase selected can include polymeric or glass beads, nitrocellulose, microparticles, microwells of a reaction tray, test tubes and magnetic beads. The signal generating compound can include an enzyme, a luminescent compound, a chromogen, a radioactive element and a chemiluminescent compound. Examples of enzymes include alkaline phosphatase, horseradish peroxidase and beta-galactosidase. Examples of enhancer compounds include biotin, anti-biotin and avidin. Examples of enhancer compounds binding members include biotin, anti-biotin and avidin.

The above methods are useful for evaluating the risk of developing diseases like cancer or an autoimmune disease due to the deleterious effects of the presence of a subgenomic HCBI, MSBI, MSSI or CMI polynucleotide sequence by itself or linked to a particular host gene or gene fragment within the patient's cells and allow taking appropriate counter measures.

Thus, the present invention also relates to an antisense oligonucleotide or iRNA specific for the MSBI1.176 or MSBI2.176 virus polynucleic acid of the invention.

The generation of suitable antisense oligonucleotides or iRNAs includes determination of a site or sites within the MSBI1.176 or MSBI2.176 polynucleic acid for the antisense interaction to occur such that the desired effect, e.g., inhibition of expression of the polypeptide, will result. A preferred intragenic site is (a) the region encompassing the translation initiation or termination codon of the open reading frame (ORF) of the gene or (b) a region of the mRNA which is a "loop" or "bulge", i.e., not part of a secondary structure. Once one or more target sites have been identified, oligonucleotides are chosen which are sufficiently complementary to the target, i.e., hybridize sufficiently well and with sufficient specificity, to give the desired effect. In the context of this invention, "hybridization" means hydrogen bonding, which may be Watson-Crick, Hoogsteen or reversed Hoogsteen hydrogen bonding, between complementary nucleoside or nucleotide bases. "Complementary" as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense compound does not need to be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired, i.e., in the case of therapeutic treatment.

"Oligonucleotide" (in particular in the context of antisense compounds) refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. While antisense oligonucleotides are a preferred form of the antisense compound, the present invention comprehends other oligomeric antisense compounds, including but not limited to oligonucleotide mimetics such as are described below. The antisense compounds in accordance with this invention comprise from about 8 to about 50 nucleobases (i.e. from about 8 to about 50 linked nucleosides). Particularly preferred antisense compounds are antisense oligonucleotides, even more preferably those comprising from about 15 to about 25 nucleobases. Antisense compounds include ribozymes, external guide sequences (EGS), oligonucleotides (oligozymes), and other short catalytic RNAs or catalytic oligonucleotides which hybridize to the target nucleic acid and inhibit its expression. The antisense compounds also include an iRNA comprising a sense sequence and an antisense sequence, wherein the sense and antisense sequences form an RNA duplex and wherein the antisense sequence comprises a nucleotide sequence sufficiently complementary to the nucleotide sequence of an MSBI1.176 or MSBI2.176 polynucleic acid of the present invention.

Alternatively, the invention provides a vector allowing to transcribe an antisense oligonucleotide of the invention, e.g., in a mammalian host. Preferably, such a vector is a vector useful for gene therapy. Preferred vectors useful for gene therapy are viral vectors, e.g. adenovirus, adeno-associated virus, herpes simplex virus, vaccinia, or, an RNA virus such as a retrovirus. Preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957. The MSBI1.176 or MSBI2.176polynucleotide sequences of the invention may also serve as a suitable vector itself, either composed solely of rearranged MSBI1.176 or MSBI2.176sequences or of chimeric MSBI1.176 or MSBI2.176host cell DNA sequences. In addition, the nucleotide sequences of the invention may be used for the construction of artificial chromosomes.

In order to achieve expression only in the target organ, the DNA sequences for transcription of the antisense oligonucleotides can be linked to a tissue specific promoter and used for gene therapy. Such promoters are well known to those skilled in the art.

Within an oligonucleotide structure, the phosphate groups are commonly referred to as forming the internucleoside backbone of the oligonucleotide. The normal linkage or backbone of RNA and DNA is a 3' to 5' phosphodiester linkage. Specific examples of preferred antisense compounds useful in the present invention include oligonucleotides containing modified backbones or non-natural internucleoside linkages. Oligonucleotides having modified backbones include those that retain a phosphorus atom in the backbone and those that do not have a phosphorus atom in the backbone. Modified oligonucleotide backbones which can result in increased stability are known to the person skilled in the art, preferably such modification is a phosphorothioate linkage.

A preferred oligonucleotide mimetic is an oligonucleotide mimetic that has been shown to have excellent hybridization properties, and is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone.

Modified oligonucleotides may also contain one or more substituted or modified sugar moieties. Preferred oligonucleotides comprise one of the following at the 2' position: OH; F; 0-, S-, or N-alkyl; 0-, S-, or N-alkenyl; 0-, S- or N-alkynyl; or 0-alkyl-O-alkyl, wherein the alkyl, alkenyl and alkynyl may be substituted or unsubstituted C₁ to C₁₀ alkyl or C₂ to C₁₀ alkenyl and alkynyl. A particularly preferred modified sugar moiety is a 2'-O-methoxyethyl sugar moiety.

Antisense-oligonucleotides of the invention may also include nucleobase modifications or substitutions. Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine etc., with 5-methylcytosine substitutions being preferred since these modifications have been shown to increase nucleic acid duplex stability.

Another modification of the oligonucleotides of the invention involves chemically linking to the oligonucleotide one or more moieties or conjugates which enhance the activity, cellular distribution or cellular uptake of the oligonucleotide. Such moieties include lipid moieties such as a cholesterol moiety, cholic acid, a thioether, a thiocholesterol, an aliphatic chain, e.g., dodecandiol or undecyl residues, a phospholipid, a polyamine or a polyethylene glycol chain, or adamantane acetic acid, a palmityl moiety, or an octadecylamine or hexylamino-carbonyl-oxycholesterol moiety.

The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers.

The present invention also relates to a pharmaceutical composition comprising an antibody or antisense oligonucleotide of the invention and a suitable excipient, diluent or carrier. Preferably, in a pharmaceutical composition, such compound as described above is combined with a pharmaceutically acceptable carrier. "Pharmaceutically acceptable" is meant to encompass any carrier, which does not interfere with the effectiveness of the biological activity of the active ingredient and that is not toxic to the host to which it is administered. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and the active compound can be administered to the subject at an effective dose.

An "effective dose" refers to an amount of the active ingredient that is sufficient to prevent the disease or to affect the course and the severity of the disease, leading to the reduction or remission of such pathology. An "effective dose" useful for treating and/or preventing these diseases or disorders may be determined using methods known to one skilled in the art.

Administration of the suitable compositions may be effected by different ways, e.g. by intravenous, intraperitoneal, oral, subcutaneous, intramuscular, topical or intradermal administration. The route of administration, of course, depends on the kind of therapy and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of therapy, general health and other drugs being administered concurrently.

In a preferred embodiment of the present disclosure, the disease that can be prevented/treated is cancer, preferably breast cancer, ovarian cancer, lung cancer, prostate cancer, pancreatic cancer, Hodgkin's disease, colorectal cancer or colon cancer or a disease of the CNS, preferably Alzheimer's disease or multiple sclerosis (MS), amyotrophic lateral sclerosis, Parkinson's disease, or transmissible spongiforme encephalopathies/Prion-linked diseases. In addition, due to a similarity of risk factors between MS and diabetes mellitus, the latter condition is also included. The terms "cancer" and "disease of the CNS" disclosed herein also comprise early stages of said diseases.

The present disclosure also relates to a vaccine for immunizing a mammal against an HCBI, MSBI, MSSI or CMI infection, comprising at least one polypeptide or HCBI, MSBI, MSSI or CMI polynucleic acid as defined above or corresponding VLP (virus-like particle) or peptide/protein/DNA complexes, in a pharmaceutically acceptable carrier. It also involves molecular and immunological tests in animals (in particular cattle) and within their products (e.g. milk and dairy products).

It may also include small chemicals for targeted therapy derived from the analysis of structural components of these agents.

A "vaccine" disclosed herein is an immunogenic composition capable of eliciting protection against HCBI, MSBI, MSSI or CMI, whether partial or complete. A vaccine may also be useful for treatment of an already infected individual, in which case it is called a therapeutic vaccine.

The term "therapeutic" disclosed herein refers to a composition capable of treating HCBI, MSBI, MSSI or CMI infection or diseases linked to this infection. The term "effective amount" disclosed herein refers to an amount of epitope-bearing polypeptide sufficient to induce an immunogenic response in the individual to which it is administered, or to otherwise detectably immunoreact in its intended system (e. g., immunoassay) . Preferably, the effective amount is sufficient to effect treatment, as defined above. The exact amount necessary will vary according to the application. For vaccine applications or for the generation of polyclonal antiserum/antibodies, for example, the effective amount may vary depending on the species, age, and general condition of the individual, the severity of the condition being treated, the particular polypeptide selected and its mode of administration, etc. Effective amounts will be found within a relatively large, non-critical range. An appropriate effective amount can be readily determined using routine experimentation. Preferred ranges of proteins disclosed herein for prophylaxis of HCBI, MSBI, MSSI or CMI caused diseases are 0.01 to 100 µg/dose, preferably 0.1 to 50 µg/dose. Several doses may be needed per individual in order to achieve a sufficient immune response and subsequent protection against an MSBI1.176 or MSBI2.176infection and an MSBI1.176 or MSBI2.176 linked disease, respectively.

Pharmaceutically acceptable carriers include any carrier that does not itself induce the production of antibodies harmful to the individual receiving the vaccine. Suitable carriers are typically large, slowly metabolized macromolecules such as proteins, polysaccharides, polylactic acids, polyglycolic acids, polymeric amino acids, and amino acid copolymers. Such carriers are well known to those of ordinary skill in the art.

Preferred adjuvants to enhance effectiveness of the composition include, but are not limited to: aluminium hydroxide (alum), N-acetyl-muramyl-L-threonyl-D-isoglutamine (thr-MDP) as found in U.S. Patent No. 4,606,918, N-acetyl-normuramyl-L-alanyl-D-isoglutamine (nor-MDP), N-acetylmuramyl-L-alanyl-D-isoglutaminyl-L-alanine-2-(1'-2'-dipaimitoyl sn-glycero-3-hydroxy-phosphoryloxy)-ethylamine (MTP-PE) and RIBI, which contains three components extracted from bacteria, monophosphoryl lipid A, trehalose dimycolate, and cell wall Skeleton (MPL +TDM + CWS) in a 2% squalene/Tween 80 emulsion. Any of the 3 components MPL, TDM or CWS may also be used alone or combined 2 by 2. Additionally, adjuvants such as Stimulon (Cambridge Bioscience, Worcester, MA) or SAF-1 (Syntex) may be used. Further, Complete Freund's Adjuvant (CFA) and Incomplete Freund's Adjuvant (IFA) may be used for non-human applications and research purposes.

The immunogenic compositions typically will contain pharmaceutically acceptable vehicles, such as water, saline, glycerol, ethanol, etc. Additionally, auxiliary substances, such as wetting or emulsifying agents, pH buffering substances, preservatives, and the like, may be included in such vehicles.

Typically, the immunogenic compositions are prepared as injectables, either as liquid solutions or suspensions. Solid forms suitable for solution in, or suspension in, liquid vehicles prior to injection may also be prepared. The preparation also may be emulsified or encapsulated in liposomes for enhanced adjuvant effect. The proteins may also be incorporated into Immune Stimulating Complexes together with saponins, for example Quil A (ISCOMS).

Immunogenic compositions used as vaccines disclosed herein comprise a "sufficient amount" or "an immunologically effective amount" of the proteins of the present invention, as well as any other of the above mentioned components, as needed. "Immunologically effective amount" means that the administration of that amount to an individual, either in a single dose or as part of a series, is effective for treatment, as defined above. This amount varies depending upon the health and physical condition of the individual to be treated, the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials. Usually the amount will vary from 0.01 to 1000 µg/dose, more particularly from 0.1 -100 µg/dose.

The following examples are intended to illustrate, but not to limit the invention. While such examples are typical of those that might be used, other methods known to those skilled in the art may alternatively be utilized.

### Example 1

### Material and Methods

### (A) Fractionation of bovine sera on density-sedimentation gradients with subsequent cloning

Initially, pools of 5 sera from a total of 120 bovine sera were subjected to Optiprep-(iodixanol)-density gradient ultracentrifugation after prior benzonase treatment to remove all free DNA and RNA (Buck et al., 2005). Protein-associated DNA was extracted from fractions (Qiagen PCR Purification Kit) and 1µl DNA/fraction subjected to RCA (rolling circle amplification) in a solution of 50µM Exo-resistant random primers (Thermo Scientific), 3.2pmol each dNTPs (Takara) and 10U phi29 polymerase (Biolabs). Restriction digested products (EcoR1 or BamH1) were separated by agarose gel electrophoresis, eluted and cloned into vector pUC19 prior to sequencing.

(B) Rolling circle amplification of DNA extracted from sera, cow milk or brain tissue: DNA was extracted by phenolchloroform from milk and post mortem brain tissue and sera from MS patients. DNA from all serum samples was extracted using the High Pure Viral Nucleic Acid Kit (Roche). RCA (rolling circle amplification) with random primers on DNA from protein-associated fractions, restriction digestion, cloning and sequencing of resulting fragments (refer above). Abutting primers used were designed either on the individual isolated DNA sequences, as well as on the replication genes of Sphinx1.76 or Sphinx2.36 and used in inverted PCR on RCA amplified DNA from single bovine sera and cow milk, as well as sera from multiple sclerosis patients and post mortem multiple sclerosis brain samples.

### Example 2

### Concept for the pathogenesis of multiple sclerosis: Isolation of circular DNA molecules (bovine agents) from bovine serum, cow milk and multiple sclerosis brain (Group 1)

The epidemiology of colon cancer suggested the involvement of an infectious factor present in red meat derived from cattle of European/Asian descent (zur Hausen, 2012; zur Hausen, 2015) and cow milk consumption has been suspected to play a role in multiple sclerosis. In attempts to isolate these putative factors, sera from 120 healthy 5-year old cows were obtained from the Veterinary Faculty of the University of Leipzig and analyzed for the presence of circular episomal DNA. Since the first isolates HCBI6.252 (Healthy Cattle Blood Isolate) (2522bp) and HCBI6.159 (1591bp) revealed a distant relationship to DNA related to sequences found in brain lesions of animals linked to prion-associated conditions (Manuelidis, 2011). The inventors concomitantly analysed 8 sera (from patients in relapse), 2 CSF and 1 PBMC from MS patients, as well as 12 biopsies from post mortem brain tissue for Sphinx-related sequences. Two circular DNA molecules related to Sphinx1.76 (1758bp acc no. HQ444404) were isolated from one MS brain sample - MSBI1.176 (Multiple Sclerosis Brain Isolate) (1766bp) and MS2.176 (1766bp). Since there is an elevated MS risk after cow milk consumption, the inventors investigated commercially available pasteurized milk for the presence of related DNA. Indeed, they isolated episomal single-stranded DNA molecules from all 4 milk samples (CMI1.252 (Cow Milk Isolate), CMI2.214, CMI3.168 and CMI4.158) (HCBI6.252 and CMI1.252 are near identical). This was taken as an indication that milk excretion of these agents is indeed occurring.

The inventors used 2 primer pairs designed on Sphinx1.76 for inverted PCR on all human and bovine samples. These primers pairs were: forward 5'-GGATTAATGCCAATGATCC-3' (nt 721-739), reverse 5'-CGAGAGAAACAGGCAAAG-3' (nt703-720) and forward 5'-GAGGACGAATTAATATTACAAGTC-3' (nt868-891), reverse 5'-TTACCAAGAAAAGCGAGAAC-3' (nt848-867). The resulting sequences are all distantly similar (ranging from 79% - 98%) to the Sphinx1.76 isolate. HCBI6.159 apparently evolved from a deletion in HCBI6.252 (deletion of nt 1129-2060 in HCBI6.252) as their overlapping sequences are identical. HCBI6.159 was isolated independently from HCBI6.252 and it is therefore highly unlikely that it is an artifact. MSBI1.176 is 98% identical to Sphinx1.76, but the nature (patterns) of the single sequence differences are such that these can be regarded as two separate agents. As the Sphinx1.76 construct was not available in the inventor's laboratory, it could not have resulted from laboratory contamination. The inventors isolated a second very distantly Sphinx1.76-related (but identical in size) circular DNA molecule MSBI2.176 from the same brain biopsy.

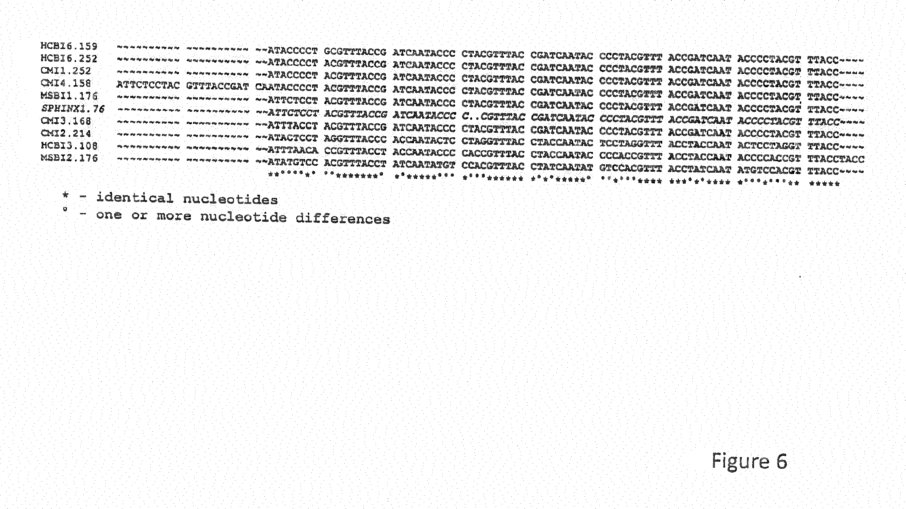

The large ORFs of the isolate of group 1 encode for replication protein (ProtSweep, del Val et al., 2007) sharing high similarity between them. Another common feature is the presence of iteron-like tandem repeats (3x22nt plus 17/18nt of the repeat in each isolate). Alignment of this repeat region indicates only single nucleotide variation in the core (Figure 6). These iteron-like repeats may constitute binding sites for Rep proteins (Chattoraj, 2000, Dziewit et al., 2013).

Nucleotide sequence accession number: The complete sequences of 8 isolates have been deposited in the EMBL Databank under the acc. no.:

| | | |
|---|---|---|
| CMI1.2522 | Acc no. | LK931487 |
| CMI2.214 | Acc no. | LK931488 |
| CMI3.168 | Acc no. | LK931489 |
| CMI4.158 | Acc no. | LK931490 |
| MSBI1.17 | Acc no. | LK931491 |
| MSBI2.176 | Acc no. | LK931492 |
| HCBI6.252 | Acc no. | LK931493 |
| HCBI6.159 | Acc no. | LK931494 |

In this context, diseases of the CNS (e.g. Multiple sclerosis MS, amyotrophic lateral sclerosis, transmissible spongiforme encephalopathies/ Prion-linked diseases, Parkinson's disease, Alzheimer disease) are also highly interesting since the similar sequences described by Manuelidis are primarily found in the CNS.

### Example 3

### Isolation of a bovine factor from bovine serum (group 2)

DNA from bovine serum was extracted followed by rolling circle amplification of the DNA. PCR amplification using abutting primers (forward (nt2313-2336) 5'CTAATGCAGATCAACACAGGGATA-3' and reverse (nt2312-2291) 5'-GAATTACAGGCTTTGCAATCTG-3') designed on the replication gene of Sphinx2.36 (2364bp, acc no. HQ444405) (Manuelidis 2011) resulted in one circular DNA isolate HCBI7.228 (2280bp, acc no. LK931498) (group 2).

### Example 4

### Isolation of sequences from bovine sera and multiple sclerosis serum distantly related to a novel group of single-stranded DNA viruses, the myco-like Gemycircularviruses (Group 3)

A large number of circular ssDNA viruses have recently been identified by metagenomic analyses (Rosario et al., 2012a). The group of Gemycircularviruses has been isolated from fungi, faeces of various animals and plants leaves (Rosario et al., 2012b; Sikorski et al. 2013). The inventors report the isolation of the genomes of 3 novel viruses distantly related to this virus group.

Two sequences HCBI8.215 (2152bp) and HCBI9.2112 (2121bp) were isolated after density gradient fractionation of bovine serum pools. Fragments obtained after restriction digest (BamH1 and EcoR1) were cloned into pUC19. Their full-length genomes were verified by inverted PCR using abutting primers. One multiple sclerosis serum isolate MSSI2.225 rescued using abutting primers and inverted PCR was related to the myco-like Gemycircularviruses isolated from cattle sera (HCBI8.215 and HCBI9.212). The inventors subsequently isolated a sequence MSBI3.224 from a post-mortem-MS affected brain tissue which proved to be identical to MSSI2.225.

The genome organization of all 3 isolates revealed a putative spliced replication protein coded for on the negative strand and the coat protein (CP) on the positive strand. The CP was arginine rich and a similarity to the TTV ORF1 protein was indicated in a DomainSweep analysis (del Val et al., 2007). Putative rolling circle motifs I, II and III and a Walker B motif of each were identified as follows: HCBI8.215 (LLTYA, HLHAFVD, YAIKD; VFDDI), for HCBI9.212 (LLKMP, HYHIYLG, YVGKD; VFDDI) and for MSSI2.225 (LLTYP, HLHAFVD, YAIKD; IFDDF). The GRS motifs were AVFDVGGFHPNISITK, TAFDYFGAHGNIKSIR and RAFDVEGCHPNVSPSR respectively. The nona-nucleotide motif for both HCBI8.215 and MSSI2.225 is (TAATGTTAT) and for HCBI9.212 (TAATATTAT).

These 3 isolates probably constitute a novel group of viruses as their capsid proteins share similarities with the ORF1 of TT viruses from the family *Anelloviridae,* and not to that of the plant virus family *Geminiviridae* as in the Gemycircularviruses. The isolation of these 3 Gemycircularvirus-related genomes directly from animal and diseased human tissue links them to have some etiological relevance for diseases such as multiple sclerosis.

| | | |
|---|---|---|
| HCBI8.215 | Acc no. | LK931483 |
| HCBI9.212 | Acc no. | LK931484 |
| MSSI2.225 | Acc no. | LK931485 |

### Example 5

### Bacterial plasmid-related circular DNA from human serum (group 4)

*Psychrobacter* species are frequently present as food contaminants and have been isolated from human tissues including brain, cerebrospinal fluid and blood. It is considered as an opportunistic human pathogen (Caspar et al., 2013; Lloyd-Puryear et al., 1991). The inventors report the isolation of a circular DNA molecule, MSSI1.162, from serum taken from a multiple sclerosis patient during relapse.

Rolling circle amplification and restriction digest were performed on DNA extracted from sera from patients with multiple sclerosis. The resulting fragment was cloned into vector pUC19. The full-length genome was verified by inverted PCR amplification using primers designed on the sequence identified initially: forward primer 5'-GACTTCTGATTGATTGATGCCTG-3' and reverse 5'-CCTGTTGAATACCGCTTAAATACT-3'. All products were sequenced by primer walking. MSSI1.162 (multiple sclerosis serum isolate) (1627bp) (Acc no. LK931486) shares 66% nucleotide similarity by BlastN analysis to the pRWF102 plasmid of the *Psychrobacter* species PRwf-1. The putative protein (321 amino acids) encoded by the large ORF shows weak similarity to replication protein family of E. coli as analysed by ProtSweep (del Val et a., 2007).

This new isolate is only distantly related to known *Psychrobacter* species and their plasmids. It may therefore represent a yet unknown human pathogen.

### Summary:

The presence of presumably infectious agents and their nucleic acids in the serum of healthy cows should imply that the same particles are also present in red meat.

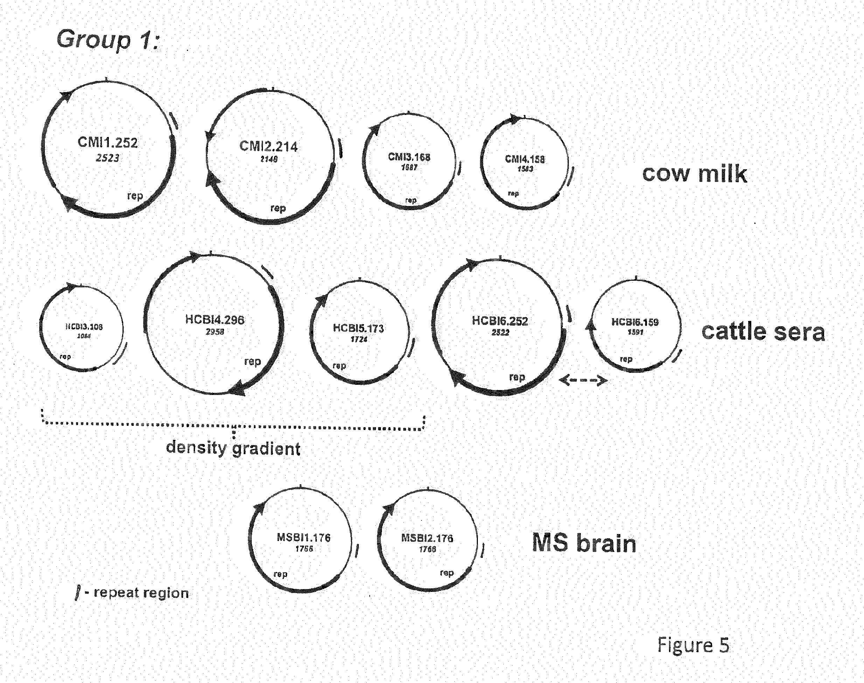

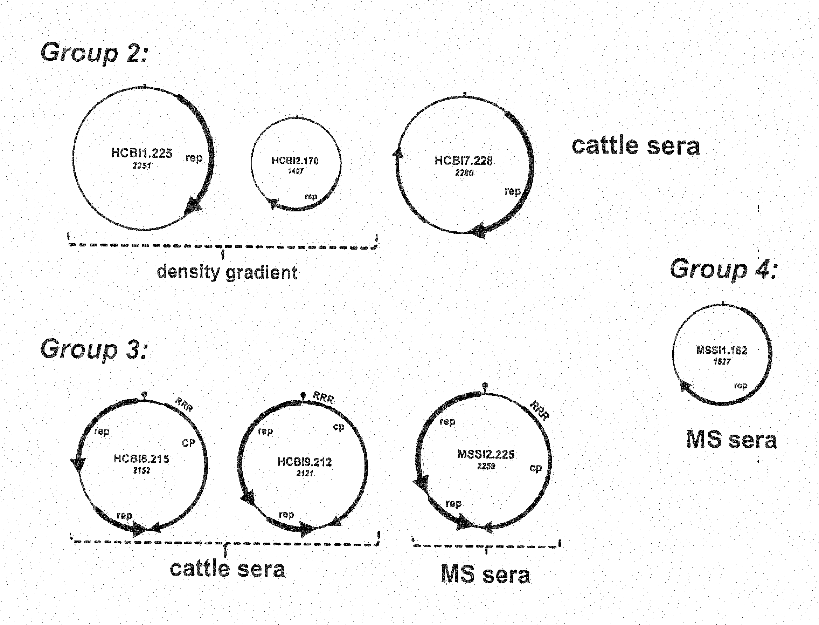

The inventors isolated 13 novel single-stranded DNA molecules from cattle serum and milk and MS brain tissue and sera. These isolates are grouped in 4 groups according to their sequence similarity to the Sphinx1.76 genome (group 1), Sphinx2.36 genome (group 2), myco-like Gemycircularviruses (group 3) and *Psychrobacter* spp. plasmid (group 4). Their genome organizations are presented in Figure 5 A, B. The main feature of all the sequences is the presence of a replication-associated protein encoding ORF.

All the isolates are presumably single-stranded DNA because of the bias of RCA towards amplification of single-stranded DNA (*del Solar et al*., *1998*). A taxonomic classification of the isolates is, at this stage, not possible. Only group 3 can be defined as clearly related to the myco-like Gemycircularviruses. This relatively novel group of viruses have been identified by metagenomics and isolated mainly from faeces of a very large variety of animals and insects, as well as plants and fungi. They share some characteristics with the Geminiviruses of plants *(Sikorski et al 2013).* Isolation from tissues of animals or humans has not yet been described. The putative replication-associated protein of all 3 isolates described in this application has an arginine-rich region with a similarity to human single-stranded TT viruses.

Infection of human cells by such agents should evoke a strong immune reaction, quite distinct from human TT viruses, where reasonable evidence for vertical transmission has been obtained (*reviewed in zur Hausen and de Villiers, 2014*). This could explain the high susceptibility to environmental factors for MS development during the first 15 years of life: primary infection may initially lead to rounds of replication and spreading of BMF probably via blood cells, eventually resulting in latent brain cell infection. This initial infection should induce an immune response, probably neutralizing the agent in subsequent rounds of infection prior to entry of the brain. The isolates reported here seem to represent excellent candidates for the postulated bovine milk factor (BMF).

A high variability in size was noted in group 1. The circular isolate HCBI6.159 seem to have evolved from HCBI6.252 through deletion of 931 nucleotides from the latter. The isolates all possess a replication gene and have an iteron-like repeat region in common (*Dziewit et al.*, *2013*). Alignment of this region between 8 isolates and Sphinxl.76 reveals a central identical core (Figure 6). Group 2 and 4 isolates do not have repeat regions.

The "Sphinx" sequences (*Manuelidis*, *2011*) show high homologies to plasmid sequences of the bacterium Acinetobacter (*Vallenet et al*., *2008; Longkumer et al*., *2013*)*.* The sequences obtained in the present invention also exhibit striking homologies to the corresponding plasmid sequences. Although a large number of plasmids have been isolated and sequenced from Acinetobacter, thus far none of them corresponded exactly to the bovine and human sequences reported in this invention. Interestingly, a group of scientists in the UK published serological data over a period of years pointing to an increased selective formation of antibodies against Acinetobacter proteins but not against other bacterial antigenes obtained from patients suffering from multiple sclerosis (see review article: *Ebringer et al., 2012*). These results could not be confirmed by the group of Chapman (*Chapman et al*., *2005*). However, it has to be stressed that the group of Chapman used a different strain of Acinetobacter (*Acinetobacter calcoaceticus*). Unequivocal results were obtained by the group of Ebringer for three strains of Acinetobacter *(Acinetobacter 1woffii, A. radioesistens* and a specific isolate, A. 11171). However, the results obtained for *A*. *junii* 17908 were less impressive and significant reactivity was hardly detectable (*Hughes et al., 2001*). These results suggest that we are dealing with strain-specific reactivities wherein this sero-reactivity is due to strain-specific plasmids exhibiting homologies to the DNA sequences obtained in the present invention.

The isolate MSSI1.162 (group 4) has similarity to a plasmid of the *Psychrobacter* spp. *Pyschrobacter* species have been considered as an opportunistic human pathogen (*Caspar et al*., *2013*) and has been isolated from a case of meningitis (*Lloyd-Puryear et al., 1991*). These bacteria have repeatedly been reported as contaminants during and after cold-storage of meat (*de Filippis et al*., *2013*) and were frequently isolated from milk and a variety of cheeses (*Coton et al., 2012*).

It is of interest to note that Manuelidis reported two "Sphinx-structures", labeled as "large" (2.36) and "small" (1.76) Sphinx. Although most of the present sequences substantially differed from her isolates, the inventors also obtained large and small Sphinx-like sequences from the same probes. Circular HCBI6.159 seems to have evolved from HCBI6.252 through a deletion of 931 nucleotides from the latter. It cannot be excluded that the other larger isolates may have smaller counterparts which were not isolated. It remains, however, to be determined whether the two structures found here persist within the same protein coat or complement each other.

The isolation of DNA of similar, in part even identical single-stranded circular nucleic acids from cattle sera, commercially available cow milk and florid MS tissues argues in favour of the concept outlined above.

### References

Buck CB, Pastrana DV, Lowy DR, Schiller JT. Efficient intracellular assembly of papillomaviral vectors. J. Virol. 2004; 78:751-757.
Chapman MD, Hughes LE, Wilson CD, Namnyak S, Thompson EJ, Giovannoni G. No evidence for production of intrathecal immunoglobulin G against Acinetobacter or Pseudomonas in multiple sclerosis. Eur Neurol. 2005; 53(1):27-31.
de Villiers EM, Borkosky SS, Kimmel R, Gunst K, and Fei JW. (2011) The diversity of Torque teno viruses: In vitro replication leads to the formation of additional replication-competent subviral Molecules. J Virol 2011; 85(14):7284-7295
Ebringer A, Hughes L, Rashid T, Wilson C. Acinetobacter Immune Responses in Multiple Sclerosis: Etiopathogenetic Role and Its Possible Use as a Diagnostic Marker. Arch Neurol. 2005; 62:33-36.
Ebringer A, Rashid T, Wilson C. The role of Acinetobacter in the pathogenesis of multiple sclerosis examined by using Popper sequences. Med Hypotheses. 2012; 78(6):763-769.
Hughes, L.E., Bonell, S., Natt, R.S., Wilson, C., Tiwana, H., Ebringer, A., Cunningham, P., Chamoun, V., Thompson, E.J., Croker, J., and Vowles, J. Antibody responses to Acinetobacter spp. and Pseudomonas aeroginosa in multiple sclerosis: prospects for diagnosis using the myelin-Acinetobacter-neurofilament antibody index. Clin. Diagn. Laboratory Immunol. 2001; 8: 1181-1188.
Longkumer T, Kamireddy S, Muthyala VR, Akbarpasha S, Pitchika GK, Kodetham G, Ayaluru M, Siddavattam D. Scientific Reports 2013; 3:2240.
Manuelidis L. Nuclease resistant circular DNAs copurify with infectivity in scrapie and CJD. J. Neurovirol. 2011; 17:131-145.
Wold, MS. Replication protein A: heterotrimeric, single-stranded DNA-binding protein required for eukaryotic DNA metabolism. Ann. Review Biochem. 1997; 66:61-92
Vallenet D, Nordmann P, Barbe V, Poirel L, Mangenot S, Bataille E, Dossat C, Gas S, Kreimeyer A, Lenoble P, Oztas S, Poulain J, Segurens B, Robert C, Abergel C, Claverie J-M, Raoult D, Medigue C, Weissenbach J, Cruveiller S. Comparative analysis of Acinetobacters: three genomes for three lifestyles. PLoS One 2008; 3(3):e1805-e1805.
Xu B, Zhi N, Hu G, Wan Z, Zheng X, Liu X, Wong S, Kajigaya S, Zhao K, Mao Q, Young NS. Hybrid DNA virus in Chinese patients with seronegative hepatitis discovered by deep sequencing. Proc Natl Acad Sci U S A. 2013; 110: 10264-9.
zur Hausen H. Red meat consumption and cancer: Reasons to suspect involvement of bovine infectious factors in colorectal cancer. Int J Cancer 2012; 130:2475-2483.
zur Hausen H., de Villiers, E.-M., Prenatal Infections with Subsequent Immune Tolerance could explain the Epidemiology of Common Childhood Cancers, World Cancer Report, IARC, Lyon, 2014, pp. 261-265.
Abdel-Hag NM, Asmar BI. Human herpes virus 6 (HHV6) infection. Indian J Pediatr. 2004; 71: 89-96.
Agranoff BW, Goldberg D. Diet and the geographical distribution of multiple sclerosis. Lancet II 1974; 1061.
Alcalde-Cabero E, Almazan-Isla J, Garcia-Merino A, de Sa J, de Pedro-Cuesta J. Incidence of multiple sclerosis among European Economic Area populations, 1985-2009: the framework for monitoring. BMC Neurol. 2013; 13: 58-80.
Alenda R, Alvarez-Lafuente R, Costa-Frossard L, Arroyo R, Mirete S, Alvarez-Cermeño JC, Villar LM. Identification of the major HHV-6 antigen recognized by cerebrospinal fluid IgG in multiple sclerosis. Eur J Neurol. 2014 Apr 12. doi: 10.1111/ene.12435.
Almohmeed YH, Avenell A, Aucott L, Vickers MA. Systematic review and meta-analysis of the sero-epidemiological association between Epstein Barr virus and multiple sclerosis. PLoS One. 2013; 8: e61110.
Angelini DF, Serafini B, Piras E, Severa M, Coccia EM, Rosicarelli B, Ruggieri S, Gasperini C, Buttari F, Centonze D, Mechelli R, Salvetti M, Borsellino G, Aloisi F, Battistini L. Increased CD8+ T cell response to Epstein-Barr virus lytic antigens in the active phase of multiple sclerosis. PLoS Pathog. 2013; 9: e1003220.
Ascherio A, Munger KL, Lunemann JD. The initiation and prevention of multiple sclerosis. Nat Rev Neurol. 2012; 8: 602-12.
Ascherio A. Environmental factors in multiple sclerosis. Expert Rev. Neurother. 2013; 13 (12s) 3-9.
Ashtari F, Jamshidi F, Shoormasti RS, Pourpak Z, Akbari M. Cow's milk allergy in multiple sclerosis patients. J Res Med Sci. 2013; 18 (Suppl 1): S62-5.
Bager P, Nielsen NM, Bihrmann K, Frisch M, Wohlfart J, Koch-Henriksen N, Melbye M, Westergaard T Sibship characteristics and risk of multiple sclerosis: a nationwide cohort study in Denmark.. Am J Epidemiol. 2006; 163: 1112-7.
Bantel-Schaal U, zur Hausen H. Adeno-associated viruses inhibit SV40 DNA amplification and replication of herpes simplex virus in SV40-transformed hamster cells. Virology 1988; 164: 64-74.
Banwell B, Bar-Or A, Cheung R, Kennedy J, Krupp LB, Becker DJ, Dosch HM; Wadsworth Pediatric Multiple Sclerosis Study Group. Abnormal T-cell reactivities in childhood inflammatory demyelinating disease and type 1 diabetes. Ann Neurol. 2008; 63: 98-111.
Baranzini SE, Mudge J, van Velkinburgh JC, Khankhanian P, Khrebtukova I, Miller NA, Zhang L, Farmer AD, Bell CJ, Kim RW, May GD, Woodward JE, Caillier SJ, McElroy JP, Gomez R, Pando MJ, Clendenen LE, Ganusova EE, Schilkey FD, Ramaraj T, Khan OA, Huntley JJ, Luo S, Kwok PY, Wu TD, Schroth GP, Oksenberg JR, Hauser SL, Kingsmore SF. Genome, epigenome and RNA sequences of monozygotic twins discordant for multiple sclerosis. Nature. 2010; 464: 1351-6.
Bauer G, Höfler P, zur Hausen H. Epstein-Barr virus induction by a serum factor. I. Induction and cooperation with additional inducers. Virology. 1982; 121: 184-94.
Bauer G, Götschl M, Höfler P.Tumor-promoting activity of Epstein-Barr-virus-inducing factor transforming growth factor type beta (EIF/TGF-beta) is due to the induction of irreversible transformation. Int J Cancer. 1991; 47: 881-8.
Ben Fredj N, Rotola A, Nefzi F, Chebel S, Rizzo R, Caselli E, Frih-Ayed M, Di Luca D, Aouni M. Identification of human herpes viruses 1 to 8 in Tunisian multiple sclerosis patients and healthy blood donors. J Neurovirol. 2012; 18 :12-19.
Beretich BD, Beretich TM. Explaining multiple sclerosis by ultraviolet exposure: a geospatial analysis. Mult Scler 2009; 15: 891-8.
Birkeland SA, Storm HH, Lamm LU, Barlow L, Blohmé I, Forsberg B, Eklund B, Fjeldborg O, Friedberg M, Frödin L, et al. Cancer risk after renal transplantation in the Nordic countries, 1964-1986. Int J Cancer. 1995 Jan 17;60(2):183-9.
Borkosky SS, Whitley C, Kopp-Schneider A, zur Hausen H, de Villiers EM. Epstein-Barr virus stimulates torque teno virus replication: a possible relationship to multiple sclerosis. PLoS One. 2012; 7: e32160.
Brecht I, Weissbrich B, Braun J, Toyka KV, Weishaupt A, Buttmann M. Intrathecal, polyspecific antiviral immune response in oligoclonal band negative multiple sclerosis. PLoS One. 2012; 7: e40431.
Buchanan R, Bonthius DJ. Measles virus and associated central nervous system sequelae. Semin Pediatr Neurol. 2012; 19: 107-14.
Butcher PJ, The distribution of multiple sclerosis in relation to the dairy industry and milk consumption. N Z Med J. 1976; 83: 427-30.
Butcher PJ. Milk consumption and multiple sclerosis-an etiological hypothesis. Med Hypotheses. 1986; 19: 169-78. Caserta MT, Mock DJ, Dewhurst S. Human herpes virus 6. Clin Infect Dis. 2001; 33: 829-33. Review.
Christensen JC. Multiple sclerosis: some epidemiological clues to etiology.
   Acta Neurol Latinoam. 1975; 21: 66-85. Review.
Christensen T. Human herpes viruses in MS. Int MS J. 2007; 14: 41-7. Review.
Conradi S, Malzahn U, Paul F, Quill S, Harms L, Then Bergh F, Ditzenbach A, Georgi T, Heuschmann P, Rosche B. Breastfeeding is associated with lower risk for multiple sclerosis. Mult Scler. 2013; 19: 553-8.
Cusick MF, Libbey JE, Fujinami RS. Multiple sclerosis: autoimmunity and viruses: Curr. Opin. Rheumatol. 2013; 25: 496-501.
Décard BF, von Ahsen N, Grunwald T, Streit F, Stroet A, Niggemeier P, Schottstedt V, Riggert J, Gold R, Chan A. Low vitamin D and elevated immunoreactivity against Epstein-Barr virus before first clinical manifestation of multiple sclerosis. J Neurool Neurosurg Psychiatry. 2012; 83: 1170-3.
Dewhurst S. Human herpes virus type 6 and human herpes virus type 7 infections of the central nervous system. Herpes. 2004; 11 Suppl 2: 105A-111A. Review.
Disanto G, Morahan JM, Barnett MH, Giovannoni G, Ramagopalan SV. The evidence for a role of B cells in multiple sclerosis. Neurology 2012; 78: 823-32.
Engels EA, Biggar RJ, Hall HI, Cross H, Crutchfield A, Finch JL, Grigg R, Hylton T, Pawlish KS, McNeel TS, Goedert JJ. Cancer risk in people infected with human immunodeficiency virus in the United States. Int J Cancer. 2008; 123: 187-94.
Ferro MT, Franciotta D, Prelle A, Bestetti A, Cinque P. Active intrathecal herpes simplex virus type 1 (HSV-1) and human herpes virus-6 (HHV-6) infection at onset of multiple sclerosis. J Neurovirol. 2012; 18: 437-4
Fotheringham J, Jacobson S. Human herpes virus 6 and multiple sclerosis: potential mechanisms for virus-induced disease. Herpes 2005; 12: 4-9.
Fraussen J, Vrolix K, Martinez-Martinez P, Losen M, De Baets MH, Stinissen P, Somers V. B cell characterization and reactivity analysis in multiple sclerosis. Autoimmun Rev. 2009; 8: 654-8.
Frolik CA, Dart LL, Meyers CA, Smith DM, Sporn MB. Purification and initial characterization of a type beta transforming growth factor from human placenta. Proc Natl Acad Sci U S A. 1983; 80: 3676-80.
Gaitán MI, Sati P, Inati SJ, Reich DS. Initial investigation of the blood-barrier in MS lesions at 7 tesla. Mult Scler. 2013; 19: 1068-73.
Geeraedts F, Wilczak N, van Binnendijk R, De Keyser J. Search for morbillivirus proteins in multiple sclerosis brain tissue. Neuroreport. 2004; 19: 15: 27-32.
Grant WB. Epidemiology of disease risks in relation to vitamin D insufficiency. Prog Biophys Mol Biol. 2006; 92: 65-79.
Grinde B. Herpes viruses: latency and reactivation - viral strategies and host response. J Oral Microbiol. 2013; doi: 10.3402/jom.v510.22766.
Guggenmos J, Schubart AS, Ogg S, Andersson M, Olsson T, Mather IH, Linington C. Antibody cross-reactivity between myelin oligodendrocyte glycoprotein and the milk protein butyrophilin in multiple sclerosis. J. Immunol. 2004; 172: 661-8.
Hawkes CH, Giovannoni G, Keir G, Cunnington M, Thompson EJ. Seroprevalence of herpes simplex virus type 2 in multiple sclerosis. Acta Neurol Scand. 2006; 114: 363-7.
Heilbronn R, Bürkle A, Stephan S, zur Hausen H. The adeno-associated virus rep gene suppresses herpes simplex virus-induced DNA amplification. J Virol. 1990a; 64: 3012-8.
Heilbronn R, Weller SK, zur Hausen H. Herpes simplex virus type 1 mutants for the origin-binding protein induce DNA amplification in the absence of viral replication. Virology. 1990b; 179: 478-81.
Heilbronn R, Albrecht I, Stephan S, Bürkle A, zur Hausen H. Human cytomegalovirus induces JC virus DNA replication in human fibroblasts. Proc Natl Acad Sci U S A. 1993; 90: 11406-10.
Henle W, Henle G. Evidence for an etiologic relation of the Epstein-Barr virus to human malignancies. Laryngoscope. 1977; 87: 467-73.
Hollis BW, Roos BA, Draper HH, Lambert PW. Vitamin D and its metabolites in human and bovine milk. J Nutr. 1981; 111: 1240-8. Holzmann C, Bauer I, Meyer P. Co-occurrence of multiple sclerosis and cancer in a BRCA1 positive family. Eur J Med Genet. 2013; 56: 577-9.
Hsiao FC, Tai AK, Deglon A, Sutkowski N, Longnecker R, Huber BT. EBV LMP-2A employs a novel mechanism to transactivate the HERV-K18 superantigen through its ITAM. Virology. 2009; 385: 261-6.
Hu Knox KK, Brewer JH, Henry JM, Harrington DJ, Carrigan DR. Human herpes virus 6 and multiple sclerosis: systemic active infections in patients with early disease. Clin Infect Dis. 2000; 31: 894-903.
Hyppönen E. Vitamin D and increasing incidence of type 1 diabetes-evidence for an association? Diabetes Obes Metab. 2010; 12: 737-43.
Isik S, Ozuguz U, Tutuncu YA, Erden G, Berker D, Acar K, Aydin Y, Akbaba G, Helvaci N, Guler S. Serum transforming growth factor-beta levels in patients with vitamin D deficiency. Eur J Intern Med. 2012; 23: 93-7.
Ito I, Waku T, Aoki M, Abe R, Nagai Y, Watanabe T, Nakajima Y, Ohkido I, Yokoyama K, Miyachi H, Shimizu T, Murayama A, Kishimoto H, Nagasawa K, Yanagisawa J. A nonclassical vitamin D receptor pathway suppresses renal fibrosis. Clin Invest. 2013; 123: 4579-94.
James E, Dobson R, Kuhle J, Baker D, Giovannoni G, Ramagopalan SV. The effect of vitamin D-related interventions on multiple sclerosis relapses: a meta-analysis. Mult Scler. 2013; 19: 1571-9.
Kakalacheva K, Münz C, Lünemann JD. Viral triggers of multiple sclerosis. Biochim Biophys Acta. 2011; 1812: 132-40.
Karner W, Bauer G. Activation of a varicella-zoster virus-specific IgA response during acute Epstein-Barr virus infection. J Med Virol. 1994; 44: 258-62.
Koch-Henriksen N, Sorensen PS. Why does the north-south gradient of incidence of multiple sclerosis seem to have disappeared on the northern hemisphere? J Neurol Sci. 2011; 311: 58-63.
Koch-Henriksen N, Stenager E, Laursen B. The use of epidemiological multiple sclerosis registers in research: the Danish MS Registry. Acta Neurol Scand Suppl. 2012; 195: 7-12.
Kotzamani D, Panou T, Mastorodemos V, Tzagournissakis M, Nikolakaki H, Spanaki C, Plaitakis A. Rising incidence of multiple sclerosis in females associated with urbanization. Neurology. 2012; 78: 1728-35.
Kurtzke JF. Multiple sclerosis in time and space-geographic clues to cause. J Neurovirol. 2000; 6 Suppl 2: S134-40.
Kurtzke JF. Epidemiology in multiple sclerosis: a pilgrim's progress. Brain. 2013; 136: 2904-17.
Kuusisto H, Hyöty H, Kares S, Kinnunen E, Elovaara I. Human herpes virus 6 and multiple sclerosis: a Finnish twin study. Mult Scler. 2008; 14: 54-8.
Latif N, Rana F, Guthrie T. Breast cancer and HIV in the era of highly active antiretroviral therapy: two case reports and review of the literature. Breast J. 2011; 17: 87-92.
Lebrun C, Debouverie M, Vermersch P, Clavelou P, Rumbach L, de Seze J, Wiertlevski S, Defer G, Gout O, Berthier F, Danzon A. Cancer risk and impact of disease-modifying treatments in patients with multiple sclerosis. Mult Scler. 2008; 14: 399-405.
Lemire JM, Adams JS, Sakai R, Jordan SC. 1 alpha,25-dihydroxyvitamin D3 suppresses proliferation and immunoglobulin production by normal human peripheral blood mononuclear cells. J Clin Invest 1984; 74: 657-61.
Libbey JE, Cusick MF, Fujinami RS. Role of pathogens in multiple sclerosis. Intern Rev Immunol. Early Online 1-18, 2013; DOI: 10.3/08830185.2013.823422
Liu H, Fu Y, Li B, Yu X, Xie J, Cheng J, Ghabrial SA, Li G, Yi X, Jiang D. Widespread horizontal gene transfer from circular single-stranded DNA viruses to eukaryotic genomes. BMC Evol Biol. 2011 Sep 26;11:276. doi: 10.1186/1471-2148-11-276.
Longkumer T, Kamireddy S, MuthyalaVR, Akbarpasha S, Pitchika GK, Kodetham G, Ayaluru M, Siddavattam D. Acinetobacter phage genome is similar to Sphinx 2.36, the circular DNA copurified with TSE infected particles. Sci Rep 2013; 3: 2240.doi: 10.1038/srep02240.
Lucchinetti C, Brück W, Parisi J, Scheithauer B, Rodriguez M, Lassmann H.Heterogeneity of multiple sclerosis lesions: implications for the pathogenesis of demyelination. Ann Neurol. 2000; 47: 707-17.
Magliozzi R, Serafini B, Rosicarelli B, Chiapetta G, Veroni C., Reynolds R, Aloisi F. B-cell enrichment and Epstein-Barr virus infection in inflammatory cortical lesions in secondary progressive multiple sclerosis. J Neuropathol Exp Neurol 2013; 72: 29-41.
Malosse D, Perron H. Correlation analysis between bovine populations, other farm animals, house pets, and multiple sclerosis prevalence, Neuroepidemiology. 1993; 12: 15-27.
Marrie RA, Wolfson C. Multiple sclerosis and varicella zoster virus infection: a review. Epidemiol Infect. 2001; 127: 315-25. Review.
Matz B, Schlehofer JR, zur Hausen H. Identification of a gene function of herpes simplex virus type 1 essential for amplification of simian virus 40 DNA sequences in transformed hamster cells. Virology. 1984; 134: 328-37.
Mesliniene S, Ramrattan L, Goldings S, Sheikh-Ali M. Role of vitamin D in the onset, progression and severity of multiple sclerosis. Endocr Pract. 2013; 19: 129-36.
Metz I, Weigand SD, Popescu BF, Frischer JM, Parisi JE, Guo Y, Lassmann H, Brück W, Lucchinetti CF. Pathologic heterogeneity persists in early active multiple sclerosis lesions. Ann Neurol. 2014 Apr 26. doi: 10.1002/ana.24163.
Midgard R, Glattre E, Grønning M, Riise T, Edland A, Nyland H. Multiple sclerosis and cancer in Norway. A retrospective cohort study. Acta Neurol Scand. 1996; 93: 411-5.
Mirandola P, Stefan A, Brambilla E, Campadelli-Fiume G, Grimaldi LM. Absence of human herpes virus 6 and 7 from spinal fluid and serum of multiple sclerosis patients. Neurology. 1999; 53: 1367-8.
Muller K, Heilmann C, Poulsen LK, Barington T, Bendtzen K. The role of monocytes and T cells in 1,25-dihydroxyvitamin D3 mediated inhibition of B cell function in vitro. Immunopharmacology 1991; 21: 121-8.
Munger KL, Chitnis, T, Frazier AL, Giovannucci E, Spiegelman D, Ascherio A. Dietary intake of vitamin D during adolescence and risk of multiple sclerosis. J Neurol. 2011a; 258: 479-85.
Munger KL Levin LI, O'Reilly EJ, Falk KI, Aschewrio A. Anti-Epstein-Barr virus antibodies as serological markers of multiple sclerosis: a prospective study among United States military personnel. Mult Scler 2011b; 17: 1185-93.
Murray TJ. An unusual occurrence of multiple sclerosis in a small rural community. Can J Neurol Sci. 1976; 3: 163-6.
Nicoll MP, ProençaJT, Efstathiou S. The molecular basis of herpes simplex virus latency. FEMS Microbiol Rev. 2012; 36: 684-705.
Nielsen NM, Rostgaard K, Rasmussen S, Koch-Henriksen N, Storm HH, Melbye M, Hjalgrim H. Cancer risk among patients with multiple sclerosis: a population-based register study. Int J Cancer. 2006; 118: 979-84.
Nora-Krukle Z, Chapenko S, Logina I, Millers A, Platkajis A, Murovska M. Human herpes virus 6 and 7 reactivation and disease activity in multiple sclerosis. Medicina (Kaunas). 2011; 47: 527-31.
Nordal HJ, Vandvik B, Norrby E. Multiple sclerosis: local syntheses of electrophoretically restricted measles, rubella, mumps and herpes simplex virus antibodies in the central nervous system. Scand J Immunol. 1978; 7: 473-9.
O'Gorman C, Lin R, Stankovich J, Broadley SA, Modelling genetic susceptibility to multiple sclerosis with family data. Neuroepidemiology. 2013; 40: 1-12.
Ohara Y. Multiple sclerosis and measles virus. Jpn J Infect Dis. 1999; 52: 198-200. Review.
Olival GS, Lima BM, Sumita LM, Serafim V, Fink MC, Nali LH, Romano CM, Thomaz RB, Cavenaghi VB, Tilbery CP, Penalva-de-Oliveira AC. Multiple sclerosis and herpes virus interaction. Arq Neuropsiquiatr. 2013; 71: 727-30.
Ongrádi J, Rajda C, Maródi CL, Csiszár A, Vecsei L. A pilot study on the antibodies to HHV-6 variants and HHV-7 in CSF of MS patients. J Neurovirol. 1999; 5: 529-32.
Opsahl ML, Kennedy PG Investigating the presence of human herpes virus 7 and 8 in multiple sclerosis and normal control brain tissue. J Neurol Sci. 2006; 240: 37-44.
Ordoñez G, Pineda B, Garcia-Navarrete R, Sotelo J. Brief presence of varicella-zoster vral DNA in mononuclear cells during relapses of multiple sclerosis. Arch Neurol. 2004; 61: 529-32.
Owens GP, Gilden D, Burgoon MP, Yu X, Bennett JL. Viruses and multiple sclerosis. Neuroscientist. 2011; 17: 659-76. Review.
Pakpoor J, Pakpoor J, Disanto G, Giovannoni G, Ramagopalan SV. Cytomegalovirus and multiple sclerosis risk. J Neurol. 2013; 260: 1658-60.
Petersen T, Moller-Larsen A, Ellermann-Eriksen S, Thiel S, Christensen T. Effects of interferon-beta therapy on elements in the antiviral immune response towards the human herpes viruses EBV, HSV, and VZV, and to the human endogenous retroviruses HERV-H and HERV-M in multiple sclerosis. J Neuroimmunol. 2012; 249: 105-8.
Pierrot-Deseilligny C, Souberbielle JC. Contribution of vitamin D insufficiency to the pathogenesis of multiple sclerosis. Ther Adv Neurol Disord. 2013; 6: 81-116.
Pisacane A, Impagliazzo N, Russo M, Valiani R, Mandarini A, Florio C, Vivo P. Breast feeding and multiple sclerosis. BMJ. 1994; 308: 1411-2.
Pohl D, Rostasy K, Jacobi C, Lange P, Nau R, Krone B, Hanefeld F. Intrathecal antibody production against Epstein-Barr and other neurotropic viruses in pediatric and adult onset multiple sclerosis. J Neurol. 2010; 257: 212-6.
Provvedini DM, Tsoukas CD, Deftos LJ, Manolagas SC. 1 alpha,25-dihydroxyvitamin D3-binding macromolecules in human B lymphocytes: effects on immunoglobulin production. J Immunol 1986; 136: 2734-40.
Rail GF. Measles virus 1998-2002: progress and controversy. Annu Rev Microbiol. 2003; 57: 343-67. Review.
Rima BK, Duprex WP. Molecular mechanisms of measles virus persistence. Virus Res. 2005; 111: 132-47. Review.
Rosecrans R, Dohnal JC. Seasonal vitamin D changes and the impact on health risk assessment. Clin Biochem, 2014; pii: S0009-9120. doi: 10.1016/j.clinbiochem.2014.02.004.
Ross RT. The varicella-zoster virus and multiple sclerosis. J Clin Epidemiol. 1998; 51: 533-5. Review.
Ruprecht K, Obojes K, Wengel V, Gronen F, Kim KS, Perron H, Schneider-Schaulies J, Rieckmann P. Regulation of human endogenous retrovirus W protein expression by herpes simplex virus type 1: implications for multiple sclerosis. J Neurovirol. 2006; 12: 65-71.
Salzer J, Nyström M, Hallmans G, Steenlund H, Wadell G, Sundström P. Epstein-Barr virus antibodies biobank samples. Mult Scler. 2013; 19: 1587-91.
Sanders V, Felisan S, Waddell A, Tourtellotte W. Detection of herpesviridae in postmortem multiple sclerosis brain tissue and controls by polymerase chain reaction. J Neurovirol. 1996; 2: 249-58.
Schlehofer JR, Gissmann L, Matz B, zur Hausen H. Herpes simplex virus-induced amplification of SV40 sequences in transformed Chinese hamster embryo cells. Int J Cancer. 1983; 32: 99-103.
Schlehofer JR, zur Hausen H. Adenovirus infection induces amplification of persistent viral DNA sequences (simian virus 40, hepatitis B virus, bovine papillomavirus) in human and rodent cells. Virus Res. 1990;17: 53-60.
Schmitt J, Schlehofer JR, Mergener K, Gissmann L, zur Hausen H. Amplification of bovine papillomavirus DNA by N-methyl-N'-nitro-N-nitrosoguanidine, ultraviolet irradiation, or infection with herpes simplex virus. Virology 1989; 172:73-81.
Scott FW. Cow milk and insulin-dependent diabetes mellitus: is there a relationship? Am J Clin Nutr. 1990; 51:489-491.
Sepcic J, Mesaros E, Materljan E, Sepic-Grahovac D. Nutritional factors and multiple sclerosis in Gorski Kotar, Croatia. Neuroepidemiology. 1993; 12: 234-40.
Simpson S Jr, Taylor B, Dwyer DE, Taylor J, Blizzard L, Ponsonby AL, Pittas F, Dwyer T, van der Mei I. Anti-HHV-6 IgG titer significantly predicts subsequent relapse risk in multiple sclerosis. Mult Scler. 2012; 18: 799-806.
Sotelo J, Corona T. Varicella zoster virus and relapsing remitting multiple sclerosis. Mult Scler Int. 2011; 2011: 214763.
Sun LM, Lin CL, Chung CJ, Liang JA, Sung FC, Kao CH. Increased breast cancer risk for patients with multiple sclerosis: a nationwide population-based cohort study. Eur J Neurol. 2013; Sep 19. doi: 10.1111/ene.12267.
Sundqvist E, Bergström T, Daialhosein H, Nyström M, Sundström P, Hillert J, Alfredsson L, Kockum I, Olsson T. Cytomegalovirus seropositivity is negatively associated with multiple sclerosis. Mult Scler. 2013 Sep 2. [Epub ahead of print].
Sundström P, Juto P, Wadell G, Hallmans G, Svenningsson A, Nyström L, Dillner J, Forsgren L. An altered immune response to Epstein-Barr virus in multiple sclerosis: a prospective study. Neurology. 2004; 62: 2277-82.
Sutkowski N, Chen G, Calderon G, Huber BT. Epstein-Barr virus latent membrane protein LMP-2A is sufficient for transactivation of the human endogenous retrovirus HERV-K18 superantigen. Virol. 2004; 78: 7852-60.
Svejgaard A. The immunogenetics of multiple sclerosis. Immunogenetics. 2008; 60: 275-86.
Tai AK, Luka J, Ablashi D, Huber BT. HHV-6A infection induces expression of HERV-K18-encoded superantigen. J Clin Virol. 2009; 46: 47-8.
Tan IL, van Schijndel RA, Pouwell PJ, van Walderveen MA, Reichenbach JR, Manoliu RA, Barkhof F. MR venography of multiple sclerosis. AJNR Am Neuroradiol. 2000; 21: 1029-42.
Tarrats R, Ordonez G, Rios C, Sotelo J. Varicella, ephemeral breastfeeding and eczema as risk factors for multiple sclerosis in Mexicans. Acta Neurol Scand. 2002; 105: 88-94..
Taus C, Pucci E, Cartechini E, Fié A, Giuliani G, Clementi M, Menzo S. Absence of HHV-6 and HHV-7 in cerebrospinal fluid in relapsing-remitting multiple sclerosis. Acta Neurol Scand. 2000; 101: 224-8.
Turcanova VL, Bundgaard B, Höllsberg P. Human herpes virus-6B induces expression of the human endogenous retrovirus K18-encoded superantigen. J Clin Virol. 2009; 46: 15-9.
Virtanen JO, Jacobson S. Viruses and multiple sclerosis. CNS Neurol Disord Drug Targets. 2012; 11: 528-44. Review.
Warren TR. The increased prevalence of multiple sclerosis among people who were born and bred in areas where goitre is endemic. Med Hypotheses. 1984; 14: 111-4.
Waubant E, Mowry EM, Krupp L, Chitnis T, Yeh EA, Kuntz N, Ness J, Belman A, Milazzo M, Gorman M, Weinstock-Guttman B, Rodriguez M, James JA. Anti-body response to common viruses and human leukocyte antigen-DRB1 in pediatric multiple sclerosis. Mult Scler. 2013; 19: 891-5.
Wikström J. Studies on the clustering of multiple sclerosis in Finland.
   Riv Patol Nerv Ment. 1976; 97: 199-204.
Winer S, Astsaturov I, Cheung RK, Schrade K, Gunaratnam L, Wood DD, Moscarello MA, O'Connor P, McKerlie C, Becker DJ, Dosch HM. T cells of multiple sclerosis patients target a common environmental peptide that causes encephalitis in mice. J Immunol. 2001; 166: 4751-6.
Wu H, Li T, Zeng M, Peng T. Herpes simplex virus type 1 infection activates the Epstein-Barr virus replicative cycle via a CREB-dependent mechanism. Cell Microbiol. 2012 ; 14: 546-59.
Yea C, Tellier R, Chong P, Westmacott G, Marrie RA, Bar-Or A, Banwell B; Canadian Pediatric Demyelinating Disease Network. Epstein-Barr virus in oral shedding of children with multiple sclerosis. Neurology. 2013; 81: 1392-9.
Zerr P, Vollath S, Palumbo-Zerr K, Tomcik M, Huang J, Distler A, Beyer C, Dees C, Gela K, Distler O, Schett G, Distler JH. Vitamin D receptor regulates TGF-β signalling in systemic sclerosis. Ann Rheum Dis. 2014 Jan 21. doi: 10.1136/annrheumdis-2013-204378.
zur Hausen H. Genital papillomavirus infections. Prog Med Virol. 1985; 32: 15-21.
zur Hausen, H. and de Villiers, E.M., Diary cattle serum and milk factors contributing to the risk of colon and breast cancers, Int. J. Cancer, 2015, Feb. 3 doi: 10.1002/ijc.29466
Zwart SR, Mehta SK, Ploutz-Snyder R, Bourbeau Y, Locke JP, Pierson DL, Smith SM. Response to vitamin D supplementation during Antarctic winter is related to BMI, and supplementation can mitigate Epstein-Barr virus reactivation. J Nutr. 2011; 141: 692-7.

### References (Examples 2-5)

Buck CB, Pastrana DV, Lowy DR, Schillier JT. 2005. Generation of HPV pseudovirions using transfection and their use in neutralization assays. Methods Mol Med 119: 445-462.
Caspar Y, Recule C, Pouzol P, Lafeuillade B, Mallaret M, Mairin M, Croize J. Psychrobacter arenosus bacteremia after blood transfusion, France. Emerging Infectious Diseases 2013; 19: 1118-1120.
Coton M, Delbes-Paus C, Irlinger F, Desmasures N, Le Fleche A, Stahl V, Montel MC, Coton E. Diversity and assessment of potential risk factors of Gram-negative isolates associated with French cheeses. Food Microbiol. 2012; 29:88-98.
De Filippis F, La Storia A, Villiani F, Ercolini D. Exploring the sources of bacterial spoilers in beefsteaks by culture-independent high-throughput sequencing. PLoS One 2013; 8:e70222.
del Solar G, Giraldo R, Ruiz-Echevarria MJ, Espinosa M, Diaz-Orejas R. Replication and control of circular bacterial plasmids. Microbiol Mol. Biol. Rev. 1998; 62:434-464.
de Villiers EM, Borkosky SS, Kimmel R, Gunst K, Fei JW. The diversity of torque teno viruses: in vitro replication leads to the formation of additional replication-competent subviral molecules. J Virol. 2011; 85: 7284-95.
de Villiers, EM, zur Hausen, H. Concept for the pathogenesis of multiple sclerosis (I): interaction of an amplifying virus and a helper-dependent bovine milk factor. (submitted)
Funk M, Gunst K, Lucansky V, Muller H, zur Hausen H, de Villiers E-M., Isolation of protein-associated circular DNA from healthy cattle serum, Genome Announcements, 2(4): e00846-14, 2014 Dziewit L, Cegielski A, Romaniuk K, Uhrynowski W, Szych A, Niesiobedzki P, Zmuda-Baranowska MJ, Zdanowski MK, Bartosik D. Plasmid diversity in arctic strains of Psychrobacter spp. Extremophiles. 2013; 17: 433-44
Ebringer A, Rashid T, Wilson C. Bovine spongiform encephalopathy, multiple sclerosis, and creutzfeldt-jakob disease are probably autoimmune diseases evoked by Acinetobacter bacteria. Ann N Y Acad Sci. 2005; 1050: 417-28.
Lloyd-Puryear M, Wallace D, Baldwin T, Hollis DG. Meningitis caused by Psychrobacter immobilis in an infant. J Clin. Microbiol. 1991; 29: 2041-2042.
Liu H, Fu Y, Li B, Yu X, Xie J, Cheng J, Ghabrial SA, Li G, Yi X, Jiang D. Widespread horizontal gene transfer from circular single-stranded DNA viruses to eukaryotic genomes. BMC Evol Biol. 2011; 11: 276. doi: 10.1186/1471-2148-11-276.
Longkumer T, Kamireddy S, MuthyalaVR, Akbarpasha S, Pitchika GK, Kodetham G, Ayaluru M, Siddavattam D. Acinetobacter phage genome is similar to Sphinx 2.36, the circular DNA copurified with TSE infected particles. Sci Rep 2013; 3: 2240.doi: 10.1038/srep02240.
Martin DP, Biagini P, Lefeuvre P, Golden M, Roumagnac P, Varsani A. Recombination in eukaryotic single stranded DNA viruses. Viruses. 2011; 3: 1699-738.
Rosario K, Duffy S, Breitbart M. 2012a. A field guide to eukaryotic circular single-stranded DNA viruses: insights gained from metagenomics. Arch. Virol. 157: 1851-1871.
Rosario K, Dayaram A, Marinov M, Ware J, Kraberger S, Stainton D, Breitbart M, Varsani A. 2012b. Diverse circular ssDNA viruses discovered in dragonflies (Odonata: Epiprocta). J Gen. Virol. 93:2668-2681.
Sikorski A, Massaro M, Kraberger S, Young LM, Smalley D, Martin DP, Varsani A. 2013. Novel myco-like DNA viruses discovered in the faecal matter of various animals. Virus Res. 177:209-216.
zur Hausen H, de Villiers EM. Prenatal Infections with Subsequent Immune Tolerance Could Explain the Epidemiology of Common Childhood Cancers. World Cancer Report 2014, IARC Lyon, pp 261-265.
de Villiers E-M, zur Hausen H. Concept for the pathogenesis of multiple sclerosis (I): Interaction of an amplifying virus and a helper-dependent bovine milk factor. (submitted).
del Val C, Ernst P, Falkenhahn M, Fladerer C, Glatting KH, Suhai S, Hotz-Wagenblatt A. 2007. ProtSweep, 2Dsweep and DomainSweep: protein analysis suite at DKFZ. Nucleic Acids Res. 35 (Web Server i-ssue):W444-450.
Chattoraj DK. 2000. Control of plasmid DNA replication by iterons: no longer paradoxical. Mol. Microbiol. 37: 467-476.
zur Hausen H. 2001. Proliferation-inducing viruses in non-permissive systems as possible causes of human cancers. Lancet 357: 381-384.
Whitley C, Gunst K, Müller H, Funk M, zur Hausen H, de Villiers E-M., Novel replication-competent circular DNA molecules from healthy cattle serum, milk and multiple sclerosis-affected human brain tissue, Genome Announcements 2(4): e00849-1-4, 2014. Lamberto I, Gunst K, Müller H, zur Hausen H, de Villiers E-M. Mycovirus-like DNA virus sequences from cattle serum, human brain and serum from multiple sclerosis patients, Genome Announcements 2(4): e00848-14, 2014
Gunst K, zur Hausen H, de Villiers E-M., Isolation of bacterial plasmid-related replication-associated circular DNA from serum sample of a multiple sclerosis patient, Genome announcement 2(4), e00847-14, 2014

## Claims

1. A MSBI1.176 or MSBI2.176 polynucleic acid comprising:
(a) a nucleotide sequence depicted in Figure 1(c) or 1(d);
(b) a nucleotide sequence being complementary to a nucleotide sequence of (a); or
(c) a nucleotide sequence which is redundant as a result of the degeneracy of the genetic code compared to any of the above-given nucleotide sequences.

2. An oligonucleotide primer comprising part of a MSBI1.176 or MSBI2.176 polynucleic acid of claim 1, said primer being capable of acting as primer for specifically sequencing or specifically amplifying the nucleic acid of certain isolates containing a nucleotide sequence as defined in claim 1

3. An oligonucleotide probe comprising part of a MSBI1.176 or MSBI2.176 polynucleic acid of claim 1, said probe being capable of acting as a hybridization probe for specific detection of the nucleic acid of certain isolates containing a nucleotide sequence of claim 1.

4. An expression vector comprising a MSBI1.176 or MSBI2.176 polynucleic acid of any one of claims 1 to 3 operably linked to prokaryotic, eukaryotic or viral transcription and translation control elements.

5. A host cell transformed or modified with an expression vector according to claim 4.

6. A polypeptide being encoded by a MSBI1.176 or MSBI2.176 polynucleic acid of claim 1.

7. An antibody or antigen binding fragment thereof specifically binding to a polypeptide of claim 6.

8. A method for the detection of a MSBI1.176 or MSBI2.176 polynucleic acid according to claim 1 in a biological sample, comprising: (a) optionally extracting sample polynucleic acid, (b) amplifying the polynucleic acid as described above with at least one primer according to claim 2, optionally a labelled primer, and (c) detecting the amplified polynucleic acid.

9. A method for the detection of a MSBI1.176 or MSBI2.176 polynucleic acid according to claim 1 in a biological sample, comprising: (a) optionally extracting sample polynucleic acid, (b) hybridizing the polynucleic acid as described above with at least one probe according to claim 3, optionally a labelled probe, and (c) detecting the hybridized polynucleic acid.

10. A method for detecting a polypeptide of claim 6 or an antibody of claim 7 present in a biological sample, comprising: (a) contacting the biological sample for the presence and/or concentration of such polypeptide or antibody as defined above, and (b) detecting the immunological complex formed between said antibody and/or said polypeptide.

11. An antisense oligonucleotide reducing or inhibiting the expression of an MSBI1.176 or MSBI2.176 polynucleic acid of claim 1 or a vector containing said antisense oligonucleotide.

12. A pharmaceutical composition comprising the antibody or antigen binding fragment thereof of claim 7 or the antisense oligonucleotide of claim 11 and a suitable pharmaceutical carrier.

## Patentansprüche

1. MSBI1.176- oder MSBI2.176-Polynukleinsäure umfassend:
(a) eine Nukleotidsequenz, die in Abbildung 1(c) oder 1(d) abgebildet ist;
(b) eine Nukleotidsequenz, die zu einer Nukleotidsequenz aus (a) komplementär ist; oder
(c) eine Nukleotidsequenz, die bedingt durch die Degeneration des genetischen Codes im Vergleich zu einer der oben angegebenen Nukleotidsequenzen redundant ist.

2. Oligonukleotidprimer umfassend einen Teil einer MSBI1.176- oder MSBI2.176-Polynukleinsäure nach Anspruch 1, wobei der Primer als Primer zur spezifischen Sequenzierung oder spezifischen Amplifizierung der Nukleinsäure von bestimmten Isolaten, die die in Anspruch 1 definierte Nukleotidsequenz enthalten, wirken kann.

3. Oligonukleotidsonde umfassend einen Teil einer MSBI1.176- oder MSBI2.176-Polynukleinsäure nach Anspruch 1, wobei die Sonde als eine Hybridisierungssonde zur spezifischen Detektion der Nukleinsäure von bestimmten Isolaten, die eine Nukleotidsequenz nach Anspruch 1 enthalten, wirken kann.

4. Expressionsvektor umfassend eine MSBI1.176- oder MSBI2.176-Polynukleinsäure nach einem der Ansprüche 1 bis 3, die funktionsfähig mit prokaryotischen, eukaryotischen oder viralen Transkriptions- und Translationskontrollelementen verbunden ist.

5. Wirtszelle, die mit einem Expressionsvektor nach Anspruch 4 transformiert oder modifiziert ist.

6. Polypeptid, das von einer MSBI1.176- oder MSBI2.176-Polynukleinsäure nach Anspruch 1 kodiert ist.

7. Antikörper oder Antigenbindefragment davon, das an ein Polypeptid nach Anspruch 6 spezifisch bindet.

8. Verfahren zur Detektion einer MSBI1.176- oder MSBI2.176-Polynukleinsäure nach Anspruch 1 in einer biologischen Probe, umfassend: (a) wahlweise Extrahieren der Polnukleinsäure der Probe, (b) Amplifizieren der Polynukleinsäure wie oben beschrieben mit mindestens einem Primer nach Anspruch 2, wahlweise mit einem markierten Primer, und (c) Detektieren der amplifizierten Polynukleinsäure.

9. Verfahren zur Detektion einer MSBI1.176- oder MSBI2.176-Polynukleinsäure nach Anspruch 1 in einer biologischen Probe, umfassend: (a) wahlweise Extrahieren der Polnukleinsäure der Probe, (b) Hybridisieren der Polynukleinsäure wie oben beschrieben mit mindestens einer Sonde nach Anspruch 3, wahlweise mit einer markierten Sonde, und (c) Detektieren der hybridisierten Polynukleinsäure.

10. Verfahren zur Detektion eines Polypeptids nach Anspruch 6 oder eines Antikörpers nach Anspruch 7, die in einer biologischen Probe vorhanden sind, umfassend: (a) Kontaktieren der biologischen Probe auf das Vorhandensein und/oder Konzentration eines oben definierten Polypeptids oder Antikörpers und (b) Detektieren des immunologischen Komplexes, der sich zwischen dem Antikörper und/oder dem Polypeptid gebildet hat.

11. Antisense-Oligonukleotid, das die Expression einer MSBI1.176- oder MSBI2.176-Polynukleinsäure nach Anspruch 1 vermindert oder hemmt, oder ein Vektor, der das Antisense-Oligonukleotid enthält.

12. Pharmazeutische Zusammensetzung umfassend den Antikörper oder das Antigenbindefragment davon nach Anspruch 7 oder das Antisense-Oligonukleotid nach Anspruch 11 und ein geeignetes pharmazeutisches Trägermolekül.

## Revendications

1. Acide polynucléique MSBI1.176 ou MSBI2.176 comprenant:
(a) une séquence nucléotidique décrite à la figure 1(c) ou 1(d);
(b) une séquence nucléotidique qui est complémentaire à la séquence nucléotidique de (a); ou
(c) une séquence nucléotidique qui est redondante du fait de la dégénérescence du code génétique en comparaison avec l'une ou l'autre des séquences nucléotidiques données ci-dessus.

2. Amorce oligonucléotidique comprenant une partie d'un acide polynucléique MSBI1.176 ou MSBI2.176 selon la revendication 1, ladite amorce étant à même d'agir comme amorce pour séquencer de manière spécifique ou amplifier de manière spécifique l'acide nucléique de certains isolats contenant une séquence nucléotidique telle que définie dans la revendication 1.

3. Sonde oligonucléotidique comprenant une partie d'un acide polynucléique MSBI1.176 ou MSBI2.176 selon la revendication 1, ladite sonde étant à même d'agir comme une sonde d'hybridation pour la détection spécifique de l'acide nucléique de certains isolats contenant une séquence nucléotidique selon la revendication 1.

4. Vecteur d'expression comprenant un acide polynucléique MSBI1.176 ou MSBI2.176 selon l'une quelconque des revendications 1 à 3 lié de manière fonctionnelle à des éléments de régulation de transcription et de translation procaryotes, eucaryotes ou viraux.

5. Cellule hôte transformée ou modifiée par un vecteur d'expression selon la revendication 4.

6. Polypeptide codé par un acide polynucléique MSBI1.176 ou MSBI2.176 selon la revendication 1.

7. Anticorps ou fragment de ce dernier se liant à l'antigène se liant de manière spécifique à un polypeptide selon la revendication 6.

8. Procédé permettant de détecter un acide polynucléique MSBI1.176 ou MSBI2.176 selon la revendication 1 dans un échantillon biologique, comprenant le fait de: (a) extraire optionnellement un échantillon d'acide polynucléique, (b) amplifier l'acide polynucléique tel que décrit ci-dessus par au moins une amorce selon la revendication 2, optionnellement une amorce étiquetée, et (c) détecter l'acide polynucléique amplifié.

9. Procédé permettant de détecter un acide polynucléique MSBI1.176 ou MSBI2.176 selon la revendication 1 dans un échantillon biologique, comprenant le fait de: (a) extraire optionnellement un échantillon d'acide polynucléique, (b) hybrider l'acide polynucléique tel que décrit ci-dessus par au moins une sonde selon la revendication 3, optionnellement une sonde étiquetée, et (c) détecter l'acide polynucléique hybridé.

10. Procédé permettant de détecter un polypeptide selon la revendication 6 ou un anticorps selon la revendication 7 présent dans un échantillon biologique, comprenant le fait de: (a) mettre en contact l'échantillon biologique pour la présence et/ou la concentration d'un tel polypeptide ou anticorps tel que défini ci-dessus, et (b) détecter le complexe immunologique formé entre ledit anticorps et/ou ledit polypeptide.

11. Oligonucléotide antisens réduisant ou inhibant l'expression d'un acide polynucléique MSBI1.176 ou MSBI2.176 selon la revendication 1 ou d'un vecteur contenant ledit oligonucléotide antisens.

12. Composition pharmaceutique comprenant l'anticorps ou le fragment de ce dernier se liant à l'antigène selon la revendication 7 ou l'oligonucléotide antisens selon la revendication 11 et un support pharmaceutique approprié.
